(19) Europäisches Patentamt
European Patent Office
Office européen des brevets

(11) EP 3 922 763 B1

(12) EUROPEAN PATENT SPECIFICATION

(45) Date of publication and mention of the grant of the patent:
**23.09.2026 Bulletin 2026/39**

(21) Application number: **20753263.1**

(22) Date of filing: **07.02.2020**

(51) International Patent Classification (IPC):
***D01F 4/00*** (2006.01) ***D01F 4/02*** (2006.01)
***C07K 14/435*** (2006.01) ***C12N 15/12*** (2006.01)
***D01D 5/06*** (2006.01)

(52) Cooperative Patent Classification (CPC):
**C07K 14/43586; C07K 14/43518; C07K 14/4741; D01D 5/06; D01F 4/00; D01F 4/02;** C07K 2319/20

(86) International application number:
**PCT/JP2020/004966**

(87) International publication number:
**WO 2020/162627 (13.08.2020 Gazette 2020/33)**

# (54) METHOD FOR MANUFACTURING ARTIFICIALLY-STRUCTURED PROTEIN FIBER

VERFAHREN ZUR HERSTELLUNG VON KÜNSTLICH STRUKTURIERTEN PROTEINFASERN

PROCÉDÉ DE FABRICATION DE FIBRE DE PROTÉINE À STRUCTURE ARTIFICIELLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.02.2019 JP 2019021015**

(43) Date of publication of application:
**15.12.2021 Bulletin 2021/50**

(73) Proprietor: **CRANE INC.**
**Tsuruoka-shi, Yamagata 997-0052 (JP)**

(72) Inventors:
- **ANDO Hirotada**
**Kariya-shi, Aichi 448-8651 (JP)**
- **ISHII Hideto**
**Tsuruoka-shi, Yamagata 997-0052 (JP)**
- **KOTAKA Koichi**
**Tsuruoka-shi, Yamagata 997-0052 (JP)**
- **SATO Ryoko**
**Tsuruoka-shi, Yamagata 997-0052 (JP)**
- **KANO Hiroshi**
**Tsuruoka-shi, Yamagata 997-0052 (JP)**
- **KOBAYASHI Jun**
**Tsuruoka-shi, Yamagata 997-0052 (JP)**
- **SATO Akito**
**Tsuruoka-shi, Yamagata 997-0052 (JP)**
- **ABE Yunosuke**
**Tsuruoka-shi, Yamagata 997-0052 (JP)**

(74) Representative: **Handley, Matthew Edward et al**
**Venner Shipley LLP**
**St James's**
**79 Oxford Street**
**Manchester M1 6EG (GB)**

(56) References cited:
**WO-A1-2011/149113** **WO-A1-2018/034111**
**WO-A1-2018/164021** **WO-A1-2018/164234**
**WO-A1-2019/151429** **JP-A- 2005 515 309**
**JP-A- 2018 512 407** **JP-A- S4 872 353**
**US-A1- 2005 054 830** **US-A1- 2018 193 524**

Remarks:
The complete document including Reference Table(s) and the Sequence Listing(s) can be downloaded from the EPO website

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 922 763 B1

## Description

### Technical Field

The present invention relates to a method for producing an artificial structural protein fiber.

### Background Art

Conventionally, silk fibroin fibers, spider silk fibroin fibers, and the like, which are regenerated silk fibers, have been known as structural protein fibers, and many production methods thereof have also been reported.

For example, a method for producing a spider silk fibroin fiber having a stress of 350 MPa or more by drawing an artificial polypeptide fiber derived from a natural spider silk fibroin structure by first stage drawing in wet heat and second stage drawing in dry heat (Patent Literature 1), a method for improving the toughness of a structural protein molded body by exposing a molded body precursor containing a structural protein to an environment of a relative humidity of 80% or more (Patent Literature 2), and the like have been proposed. However, fibers having a small diameter are not obtained.

Further, for example, a method has been proposed in which a mixed aqueous solution of silk fibroin and PEO is discharged from a spinneret to which a voltage is applied and electrospun to obtain a blended fiber of silk fibroin and PEO having an average fiber diameter of less than 800 nm (Patent Literature 3). However, in addition to the need for special equipment for electrospinning, it has been difficult to obtain a fiber having a small diameter and having a sufficient stress.

Patent Literature 4 discloses a spinning process in which drawing is carried out in a drawing bath after the coagulation bath.

Patent Literature 5 discloses a process, wherein a pulling ratio of 0.5 or 0.6 is applied in the coagulating bath.

### Citation List

#### Patent Literature

Patent Literature 1: JP 5540166 B
Patent Literature 2: WO 2017/131196 A
Patent Literature 3: JP 2014-138877 A
Patent Literature 4: WO 2018/164021 A1
PatentLiterature 5: WO 2018/034111 A1

### Summary of Invention

#### Technical Problem

For development in various applications, a structural protein fiber having a small diameter and having a stress equal to or higher than that of the related art has been required.

#### Solution to Problem

The present invention relates to the method disclosed in claims 1 to 12.

#### Advantageous Effects of Invention

According to the present invention, it is possible to provide a method for producing an artificial structural protein fiber having a stress equal to or higher than that of the related art and having a small diameter by a simple method not requiring special equipment.

### Brief Description of Drawings

Fig. 1 is a schematic view illustrating an example of a domain sequence of a spider fibroin.
Fig. 2 is a schematic view illustrating an example of a domain sequence of a spider fibroin.

Fig. 3 is a schematic view illustrating an example of a domain sequence of a spider fibroin.
Fig. 4 is an explanatory view illustrating an example of a spinning apparatus for producing a protein fiber.

Description of Embodiments

Hereinafter, embodiments of the present invention will be described in detail. However, the present invention is not limited to the following embodiments.

[Method for producing artificial structural protein fiber]

A method for producing an artificial structural protein fiber of the present invention is a method for producing an artificial structural protein fiber by a wet spinning method, the method including a coagulation step of discharging a spinning dope containing an artificial structural protein and an organic solvent from a spinneret into a coagulation liquid to coagulate the artificial structural protein, wherein a spinning draft (bath draft) in the coagulation step is more than 0.4 and 20 or less.

<Spinning dope>

The spinning dope (also referred to as "dope solution") according to the present embodiment contains an artificial structural protein and an organic solvent.

(Artificial structural protein)

The artificial structural protein of the present embodiment is an artificially produced structural protein, and is not a natural protein or a protein obtained by purifying the natural protein. The structural protein means a protein that forms or maintains its structure, form, and the like in vivo. The method for artificially producing a structural protein is not particularly limited. The protein may be those produced by microorganisms or the like by a gene recombination technology, or may be those produced through synthesis.

The artificial structural protein according to the present embodiment may satisfy any one of the following (1) and (2).

(1) The number of amino acid residues is 150 or more, the content of an alanine residue is 12 to 40%, and the content of a glycine residue is 11 to 55%.
(2) The total of the content of at least one type of amino acid residue selected from the group consisting of serine, threonine, and tyrosine, the content of an alanine residue, and the content of a glycine residue is 56% or more.

In the present specification, the term "content of an alanine residue" is a value represented by the following equation.

Content of alanine residue= (number of alanine residues contained in artificial structural protein/number of all amino acid residues of artificial structural protein) $\times$ 100 (%)

In addition, the content of the glycine reside, the content of the serine residue, the content of the threonine residue, and the content of the tyrosine residue have the same meaning as those obtained by replacing the alanine residue with the glycine residue, the serine residue, the threonine residue, and the tyrosine residue, respectively, in the above equation.

In the artificial structural protein satisfying the above (1), the number of amino acid residues may be 150 or more. The number of amino acid residues may be, for example, 200 or more or 250 or more, and is preferably 300 or more, 350 or more, 400 or more, 450 or more, or 500 or more.

The content of the alanine residue of the artificial structural protein satisfying the above (1) may be 12 to 40%. The content of the alanine residue may be, for example, 15 to 40%, 18 to 40%, 20 to 40%, or 22 to 40%.

The content of the glycine residue of the artificial structural protein satisfying the above (1) may be 11 to 55%. The content of the glycine residue may be, for example, 11% to 55%, 13% to 55%, 15% to 55%, 18% to 55%, 20% to 55%, 22% to 55%, or 25% to 55%.

In the artificial structural protein satisfying the above (2), the total content of the content of at least one type of amino acid residue selected from the group consisting of serine, threonine, and tyrosine (that is, any one of the content of the serine residue, the content of the threonine residue, the content of the tyrosine residue, the total of the content of the serine residue and the content of the threonine residue, the total of the content of the serine residue and the content of the tyrosine residue, the total of the content of the threonine residue and the content of the tyrosine residue, and the total of the content of the serine residue, the content of the threonine residue, and the content of the tyrosine residue), the content of

the alanine residue, and the content of the glycine residue may be 56% or more. The total content may be, for example, 57% or more, 58% or more, 59% or more, or 60% or more. The upper limit of the total content is not particularly limited, but may be, for example, 90% or less, 85% or less, or 80% or less.

In one embodiment, the total of the content of the serine residue, the content of the threonine residue, and the content of the tyrosine residue of the artificial structural protein satisfying (2) may be 4% or more, 4.5% or more, 5% or more, 5.5% or more, 6% or more, 6.5% or more, or 7% or more. The total of the content of the serine residue, the content of the threonine residue, and the content of the tyrosine residue may be, for example, 35% or less, 33% or less, 30% or less, 25% or less, or 20% or less.

The artificial structural protein according to the present embodiment preferably satisfies both of the above (1) and (2). Accordingly, the effect of the present invention is more remarkably exhibited.

In the artificial structural protein according to the present embodiment, the distribution of serine residues, threonine residues, or tyrosine residues is mean, and the total content of the serine residue, threonine residue, and tyrosine residue among optional 20 continuous amino acid residues may be 5% or more, 10% or more, or 15% or more, and may be 50% or less, 40% or less, 30% or less, or 20% or less.

The artificial structural protein according to one embodiment may have a repeating sequence. That is, the artificial structural protein according to the present embodiment may have a plurality of amino acid sequences (repeating sequence units) having high sequence identity in the artificial structural protein. The amino acid sequence of the repeating sequence unit is not particularly limited as long as the entire artificial structural protein satisfies (1) or (2) described above. The number of amino acid residues of the repeating sequence unit is preferably 6 to 200. The sequence identity between the repeating sequence units may be, for example, 85% or more, 90% or more, 95% or more, 96% or more, 97% or more, 98% or more, or 99% or more.

The artificial structural protein according to one embodiment may include an $(A)_n$ motif. In the present specification, the $(A)_n$ motif means an amino acid sequence mainly consisting of alanine residues. The number of amino acid residues of the $(A)_n$ motif may be 2 to 27, and may be an integer of 2 to 20, 2 to 16, or 2 to 12. The proportion of the number of alanine residues in the total number of amino acid residues in the $(A)_n$ motif may be 40% or more, 60% or more, 70% or more, 80% or more, 83% or more, 85% or more, 86% or more, 90% or more, 95% or more, or 100% (which means that the $(A)_n$ motif consists of only alanine residues).

In one embodiment, the $(A)_n$ motifs may be included in the repeating sequence unit. The $(A)_n$ motif mainly contains alanine residues, and thus tends to have an $\alpha$-helix structure or a $\beta$-sheet structure. When the $(A)_n$ motif is included in the repeating sequence unit, the artificial structural protein according to the present embodiment has these repeating secondary structures. Therefore, when the artificial structural protein is in the form of a fiber, the artificial structural protein is expected to exhibit high stress due to these secondary structures.

Examples of the structural protein include spider silk protein (spider silk fibroin, for example), silk protein (silk fibroin), collagen protein, resilin protein, elastin protein, and keratin protein.

The spider silk protein includes naturally occurring spider silk protein and modified spider silk protein (hereinafter, also referred to as "modified spider silk fibroin" or simply referred to as "modified fibroin"). The term "naturally occurring spider silk protein" as used herein means a spider silk protein having the same amino acid sequence as a naturally occurring spider silk protein (spider silk fibroin, for example), and the "modified spider silk protein" or the "modified spider silk fibroin" means a spider silk protein having an amino acid sequence different from that of the naturally occurring spider silk protein.

Examples of the naturally occurring spider silk protein include spider fibroins produced by spiders, such as major dragline silk proteins, flagelliform silk proteins, and minor ampullate gland proteins. The major dragline silk has a repetitive region composed of crystal regions and noncrystal regions (also referred to as amorphous region), and therefore has high stress and stretchability. The spider flagelliform silk does not have crystal regions, but have a repetitive region composed of amorphous regions. The flagelliform silk has high stretchability, although its stress is inferior to that of the major dragline silk.

The major dragline silk protein is produced in the major ampullate glands of spiders, and has a feature of being excellent in toughness. Examples of the major dragline silk protein include major ampullate spidroins MaSp1 and MaSp2 derived from *Nephila clavipes* and ADF3 and ADF4 derived from *Araneus diadematus.* ADF3 is one of the two major dragline silk proteins of *Araneus diadematus.* The spider silk protein may be a spider silk protein derived from these dragline silk proteins. The spider silk protein derived from ADF3 is relatively easy to synthesize and has excellent characteristics in terms of strength elongation and toughness.

The flagelliform silk protein is produced in flagelliform glands of spiders. Examples of the flagelliform silk protein include flagelliform silk proteins derived from *Nephila clavipes*.

Examples of the spider fibroin produced by spiders include spider silk proteins produced by spiders belonging to the genus *Araneus* such as *Araneus ventricosus, Araneus diadematus, Araneus pinguis, Araneus pentagrammicus,* and *Araneus nojimai*; spiders belonging to the genus *Neoscona* such as *Neoscona scylla, Neoscona nautica, Neoscona adianta,* and *Neoscona scylloides*; spiders belonging to the genus *Pronus* such as *Pronous minutes*; spiders belonging to

the genus *Cyrtarachne* such as *Cyrtarachne bufo* and *Cyrtarachne inaequalis*; spiders belonging to the genus *Gasteracantha* such as *Gasteracantha kuhli* and *Gasteracantha mammosa*; spiders belonging to the genus *Ordgarius* such as *Ordgarius hobsoni* and *Ordgarius sexspinosus*; spiders belonging to the genus *Argiope* such as *Argiope amoena, Argiope minuta,* and *Argiope bruennich*; spiders belonging to the genus *Arachnura* such as *Arachnura logio*; spiders belonging to the genus *Acusilas* such as *Acusilas coccineus*; spiders belonging to the genus *Cytophora* such as *Cyrtophora moluccensis, Cyrtophora exanthematica,* and *Cyrtophora unicolor*; spiders belonging to the genus *Poltys* such as *Poltys illepidus*; spiders belonging to the genus *Cyclosa* such as *Cyclosa octotuberculata, Cyclosa sedeculata, Cyclosa vallata,* and *Cyclosa atrata*; and spiders belonging to the genus *Chorizopes* such as *Chorizopes nipponicus*; and spider silk proteins produced by spiders belonging to the genus *Tetragnatha* such as *Tetragnatha praedonia, Tetragnatha maxillosa, Tetragnatha extensa,* and *Tetragnatha squamata*; spiders belonging to the genus *Leucauge* such as *Leucauge magnifwca, Leucauge blanda,* and *Leucauge subblanda*; spiders belonging to the genus *Nephila* such as *Nephila clavate* and *Nephila pilipes*; spiders belonging to the genus *Menosira* such as *Menosira ornata*; spiders belonging to the genus *Dyschiriognatha* such as *Dyschiriognatha tenera*; spiders belonging to the genus *Latrodectus* such as *Latrodectus mactans, Latrodectus hasseltii, Latrodectus geometricus,* and *Latrodectus tredecimguttatus*; and spiders belonging to the family *Tetragnathidae* such as spiders belonging to the genus *Euprosthenops.*

More specific examples of the spider silk protein produced by spiders include fibroin-3 (adf-3) [derived from *Araneus diadematus*] (GenBank Accession No. AAC47010 (amino acid sequence), U47855 (base sequence)), fibroin-4 (adf-4) [derived from *Araneus diadematus*] (GenBank Accession No. AAC47011 (amino acid sequence), U47856 (base sequence)), dragline silk protein spidroin 1 [derived from *Nephila clavipes*] (GenBank Accession No. AAC04504 (amino acid sequence), U37520 (base sequence)), major ampullate spidroin 1 [derived from *Latrodectus hesperus*] (GenBank Accession No. ABR68856 (amino acid sequence), EF595246 (base sequence)), dragline silk protein spidroin 2 [derived from *Nephila clavata*] (GenBank Accession No. AAL32472 (amino acid sequence), AF441245 (base sequence)), major ampullate spidroin 1 [derived from *Euprosthenops australis*] (GenBank Accession No. CAJ00428 (amino acid sequence), AJ973155 (base sequence)), and major ampullate spidroin 2 [*Euprosthenops australis*] (GenBank Accession No. CAM32249.1 (amino acid sequence), AM490169 (base sequence)), minor ampullate silk protein 1 [*Nephila clavipes*] (GenBank Accession No. AAC14589.1 (amino acid sequence)), minor ampullate silk protein 2 [*Nephila clavipes*] (GenBank Accession No. AAC14591.1 (amino acid sequence)), and minor ampullate spidroin-like protein [*Nephilengys cruentata*] (GenBank Accession No. ABR37278.1 (amino acid sequence).

The spider silk protein may be, for example, a protein including a domain sequence represented by Formula 1: $[(A)_n$ motif-REP$]_m$ or Formula 2: $[(A)_n$ motif-REP$]_m$-$(A)_n$ motif. In the spider silk protein, an amino acid sequence (N-terminal sequence and C-terminal sequence) may be further added to either or both of the N-terminal side and C-terminal side of the domain sequence. The N-terminal sequence and the C-terminal sequence are not limited thereto, but, typically are regions having no repetitions of amino acid motifs characterized in fibroin, and each consist of amino acids of approximately 100 residues.

The term "domain sequence" as used herein is an amino acid sequence that produces a crystal region (typically, corresponding to the $(A)_n$ motif of the amino acid sequence) and an amorphous region (typically, corresponding to the REP of the amino acid sequence) specific to fibroin, and means an amino acid sequence represented by Formula 1: $[(A)_n$ motif-REP$]_m$ or Formula 2: $[(A)_n$ motif-REP$]_m$-$(A)_n$ motif. Here, the $(A)_n$ motif represents an amino acid sequence mainly consisting of alanine residues, and the number of amino acid residues is 2 to 27. The number of amino acid residues of the $(A)_n$ motif may be 2 to 20, 4 to 27, 4 to 20, 8 to 20, 10 to 20, 4 to 16, 8 to 16, or 10 to 16. The proportion of the number of alanine residues in the total number of amino acid residues in the $(A)_n$ motif may be 40% or more, 60% or more, 70% or more, 80% or more, 83% or more, 85% or more, 86% or more, 90% or more, 95% or more, or 100% (which means that the $(A)_n$ motif consists of only alanine residues). At least seven of a plurality of $(A)_n$ motifs in the domain sequence may consist of only alanine residues. The REP represents an amino acid sequence consisting of 2 to 200 amino acid residues. The REP may be an amino acid sequence consisting of 10 to 200 amino acid residues. m represents an integer of 2 to 300, and may be an integer of 10 to 300. A plurality of $(A)_n$ motifs may be the same amino acid sequences or different amino acid sequences. A plurality of REPs may be the same amino acid sequences or different amino acid sequences.

The fibroin is not particularly limited as long as it satisfies the above (1) or (2). Specific examples of the fibroin include fibroin (modified fibroin) shown in Table 1 below.

[Table 1]

| | Alanine residue content [%] | Glycine residue content [%] | Serine residue content [%] | Threonine residue content [%] | Tyrosine residue content [%] |
|---|---|---|---|---|---|
| PRT799 (SEQ ID NO: 15) | 19.8 | 31 | 9.3 | 0 | 7.0 |
| PRT313 (SEQ ID NO: 16) | 25.7 | 36 | 8.9 | 0 | 6.4 |

(continued)

| | Alanine residue content [%] | Glycine residue content [%] | Serine residue content [%] | Threonine residue content [%] | Tyrosine residue content [%] |
|---|---|---|---|---|---|
| PRT918 (SEQ ID NO: 39) | 19.5 | 30.9 | 9. 7 | 0 | 7.0 |
| PRT966 (SEQ ID NO: 44) | 19.7 | 31.2 | 9.4 | 0 | 7.1 |

The modified spider silk fibroin (modified fibroin) may be, for example, those obtained by modifying an amino acid sequence derived from naturally occurring spider fibroin (for example, those obtained by modifying the gene sequence of cloned naturally occurring spider fibroin to modify the amino acid sequence thereof), or may be those obtained by being artificially designed and synthesized rather than depending on naturally occurring spider fibroin (for example, those having a desired amino acid sequence obtained by chemically synthesizing a nucleic acid encoding the designed amino acid sequence).

The modified fibroin can be obtained by, for example, performing modification of the amino acid sequence corresponding to, for example, substitution, deletion, insertion, and/or addition of one or a plurality of amino acid residues, with respect to the gene sequence of cloned naturally occurring spider fibroin. The substitution, deletion, insertion, and/or addition of amino acid residues can be performed by a method known to those skilled in the art such as site-directed mutagenesis. Specifically, the modification may be performed according to a method described in literatures such as Nucleic Acid Res.10, 6487 (1982), and Methods in Enzymology, 100, 448 (1983).

Specific examples of the modified fibroin include a modified fibroin derived from major dragline silk proteins produced in major ampullate glands of spiders (first modified fibroin), a modified fibroin having a reduced content of glycine residue (second modified fibroin), a modified fibroin having a reduced content of $(A)_n$ motif (third modified fibroin), a modified fibroin having a reduced content of glycine residue and a reduced content of $(A)_n$ motif (fourth modified fibroin), a modified fibroin having a domain sequence including a region with locally high hydropathy index (fifth modified fibroin), and a modified fibroin having a domain sequence having a reduced content of glutamine residue (sixth modified fibroin).

The modified fibroin derived from major dragline silk proteins produced in major ampullate glands of spiders (first modified fibroin) includes a protein including a domain sequence represented by Formula 1: $[(A)_n \text{ motif-REP}]_m$. In the first modified fibroin, in Formula 1, n is preferably an integer of 3 to 20, more preferably an integer of 4 to 20, still more preferably an integer of 8 to 20, even still more preferably an integer of 10 to 20, still further preferably an integer of 4 to 16, particularly preferably an integer of 8 to 16, and most preferably an integer of 10 to 16. In the first modified fibroin, in Formula 1, the number of amino acid residues constituting the REP is preferably 10 to 200, more preferably 10 to 150, still more preferably 20 to 100, and even still more preferably 20 to 75. In the first modified fibroin, the total number of glycine residues, serine residues, and alanine residues included in the amino acid sequence represented by Formula 1: $[(A)_n \text{ motif-REP}]_m$ is preferably 40% or more, more preferably 60% or more, and still more preferably 70% or more, relative to the total number of amino acid residues.

The first modified fibroin may be a protein which includes units of an amino acid sequence represented by Formula 1: $[(A)_n \text{ motif-REP}]_m$ and whose C-terminal sequence is an amino acid sequence set forth in any of SEQ ID NO: 1 to 3 or an amino acid sequence having a homology of 90% or more with the amino acid sequence set forth in any of SEQ ID NO: 1 to 3.

The amino acid sequence set forth in SEQ ID NO: 1 is identical to an amino acid sequence consisting of 50 amino acid residues of the C-terminal of an amino acid sequence of ADF3 (GI: 1263287, NCBI). The amino acid sequence set forth in SEQ ID NO: 2 is identical to an amino acid sequence set forth in SEQ ID NO: 1 in which 20 amino acid residues have been removed from the C-terminal. The amino acid sequence set forth in SEQ ID NO: 3 is identical to an amino acid sequence set forth in SEQ ID NO: 1 in which 29 amino acid residues have been removed from the C-terminal.

More specific examples of the first modified fibroin include modified fibroins including (1-i) the amino acid sequence set forth in SEQ ID NO: 4, or (1-ii) an amino acid sequence having a sequence identity of 90% or more with the amino acid sequence set forth in SEQ ID NO: 4. The sequence identity is preferably 95% or more.

The amino acid sequence set forth in SEQ ID NO: 4 is an amino acid sequence obtained by the following mutation: in an amino acid sequence of ADF3 in which an amino acid sequence (SEQ ID NO: 5) consisting of a start codon, a His 10-tag and an HRV3C protease (Human rhinovirus 3C protease) recognition site is added to the N-terminal, the 1st to 13th repetitive regions are about doubled and the translation ends at the 1154th amino acid residue. The C-terminal amino acid sequence of the amino acid sequence set forth in SEQ ID NO: 4 is identical to the amino acid sequence set forth in SEQ ID NO: 3.

The modified fibroin of (1-i) may consist of the amino acid sequence set forth in SEQ ID NO: 4.

The modified fibroin having a reduced content of glycine residue (second modified fibroin) has a domain sequence having an amino acid sequence having a reduced content of glycine residue, as compared to naturally occurring

spider fibroin. The second modified fibroin may have an amino acid sequence corresponding to an amino acid sequence in which at least one or a plurality of glycine residues in REP are substituted with another amino acid residue, as compared to naturally occurring spider fibroin.

The second modified fibroin may have a domain sequence having an amino acid sequence corresponding to an amino acid sequence in which, in at least one motif sequence selected from GGX and GPGXX (where G represents a glycine residue, P represents a proline residue, and X represents an amino acid residue other than glycine) in REP, one glycine residue in at least one or a plurality of the motif sequences is substituted with another amino acid residue, as compared to naturally occurring spider fibroin.

In the second modified fibroin, the proportion of the motif sequence in which the glycine residue has been substituted with another amino acid residue may be 10% or more relative to the entire motif sequence.

The second modified fibroin includes a domain sequence represented by Formula 1: $[(A)_n \text{ motif-REP}]_m$, and may have an amino acid sequence in which z/w is 30% or more, 40% or more, 50% or more, or 50.9% or more in a case where the total number of amino acid residues in the amino acid sequence consisting of XGX (where X represents an amino acid residue other than glycine) contained in all REPs in a sequence excluding the sequence from the $(A)_n$ motif located at the most C-terminal side to the C-terminal of the domain sequence from the domain sequence is defined as z, and the total number of amino acid residues in a sequence excluding the sequence from the $(A)_n$ motif located at the most C-terminal side to the C-terminal of the domain sequence from the domain sequence is defined as w. The number of alanine residues relative to the total number of amino acid residues in the $(A)_n$ motif may be 83% or more, preferably 86% or more, more preferably 90% or more, still more preferably 95% or more, and even still more preferably 100% (which means that the $(A)_n$ motif consists of only alanine residues).

The second modified fibroin is preferably one in which the content ratio of the amino acid sequence consisting of XGX is increased by substituting one glycine residue of the GGX motif with another amino acid residue. In the second modified fibroin, the content ratio of the amino acid sequence consisting of GGX in the domain sequence is preferably 30% or less, more preferably 20% or less, still more preferably 10% or less, even still more preferably 6% or less, still further preferably 4% or less, and particularly preferably 2% or less. The content ratio of the amino acid sequence consisting of GGX in the domain sequence can be calculated by the same method as the calculation method of the content ratio (z/w) of the amino acid sequence consisting of XGX described below.

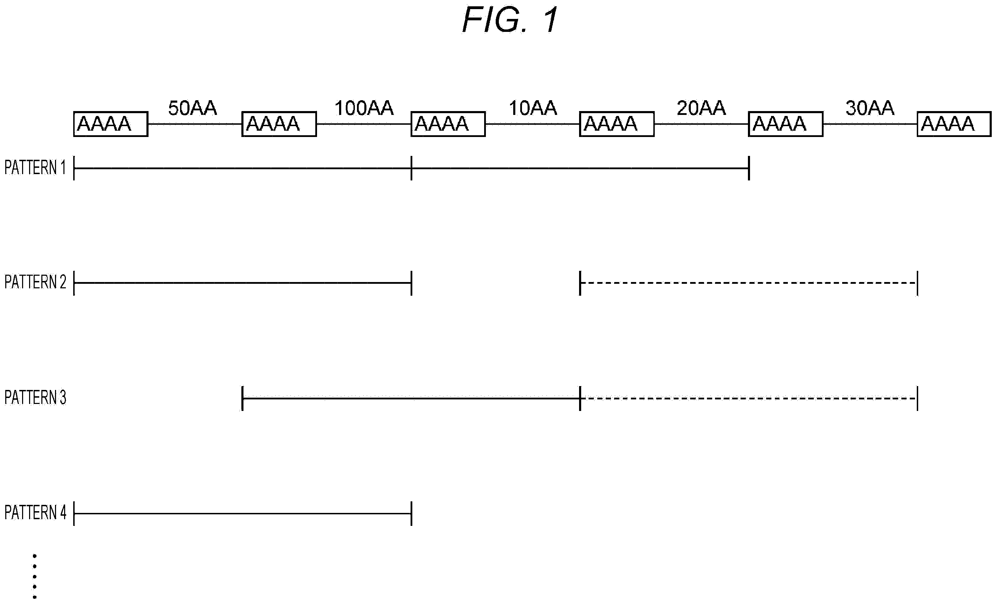

The calculation method of z/w will be described in more detail. First, in a spider fibroin (modified fibroin or naturally occurring spider fibroin) including a domain sequence represented by Formula 1: $[(A)_n \text{ motif-REP}]_m$, an amino acid sequence consisting of XGX is extracted from all REPs contained in a sequence excluding the sequence from the $(A)_n$ motif located at the most C-terminal side to the C-terminal of the domain sequence from the domain sequence. The total number of amino acid residues constituting XGX is z. For example, in a case where 50 amino acid sequences consisting of XGX are extracted (there is no overlap), z is $50 \times 3 = 150$. Also, for example, in a case where X (central X) contained in two XGXs exists as in a case of the amino acid sequence consisting of XGXGX, z is calculated by subtracting the overlapping portion (in a case of XGXGX, it is 5 amino acid residues). w is the total number of amino acid residues contained in a sequence excluding the sequence from the $(A)_n$ motif located at the most C-terminal side to the C-terminal of the domain sequence from the domain sequence. For example, in a case of the domain sequence shown in Fig. 1, w is $4 + 50 + 4 + 100 + 4 + 10 + 4 + 20 + 4 + 30 = 230$ (excluding the $(A)_n$ motif located at the most C-terminal side). Next, z/w (%) can be calculated by dividing z by w.

In the second modified fibroin, z/w is preferably 50.9% or more, more preferably 56.1% or more, still more preferably 58.7% or more, even still more preferably 70% or more, and still further preferably 80% or more. The upper limit of z/w is not particularly limited, but may be 95% or less, for example.

The second modified fibroin can be obtained by, for example, substituting and modifying at least a part of a base sequence encoding a glycine residue from the gene sequence of cloned naturally occurring spider fibroin so as to encode another amino acid residue. At this time, one glycine residue in the GGX motif and GPGXX motif may be selected as a glycine residue to be modified, and substitution may be performed so that z/w is 50.9% or more. Alternatively, the second modified fibroin can also be obtained by, for example, designing an amino acid sequence satisfying each of the above embodiments from the amino acid sequence of naturally occurring spider fibroin, and chemically synthesizing a nucleic acid encoding the designed amino acid sequence. In any case, in addition to modification corresponding to substitution of a glycine residue in REP with another amino acid residue from the amino acid sequence of naturally occurring spider fibroin, modification of the amino acid sequence corresponding to substitution, deletion, insertion, and/or addition of one or a plurality of amino acid residues may be further performed.

The above-described another amino acid residue is not particularly limited as long as it is an amino acid residue other than a glycine residue, but is preferably a hydrophobic amino acid residue such as a valine (V) residue, a leucine (L) residue, an isoleucine (I) residue, a methionine (M) residue, a proline (P) residue, a phenylalanine (F) residue, and a tryptophan (W) residue; and a hydrophilic amino acid residue such as a glutamine (Q) residue, an asparagine (N) residue, a serine (S) residue, a lysine (K) residue, and a glutamic acid (E) residue. Among them, a valine (V) residue, a leucine (L) residue, an isoleucine (I) residue, and a glutamine (Q) residue are more preferable, and a glutamine (Q) residue is still more

preferable.

More specific examples of the second modified fibroin include modified fibroins including (2-i) the amino acid sequence set forth in SEQ ID NO: 6, SEQ ID NO: 7, SEQ ID NO: 8, or SEQ ID NO: 9, or (2-ii) an amino acid sequence having a sequence identity of 90% or more with the amino acid sequence set forth in SEQ ID NO: 6, SEQ ID NO: 7, SEQ ID NO: 8, or SEQ ID NO: 9.

The modified fibroin of (2-i) will be described. The amino acid sequence set forth in SEQ ID NO: 6 is an amino acid sequence obtained by substituting all GGX in REP of the amino acid sequence set forth in SEQ ID NO: 10 corresponding to naturally occurring spider fibroin with GQX. The amino acid sequence set forth in SEQ ID NO: 7 is an amino acid sequence obtained by deleting the $(A)_n$ motif every other two positions from the N-terminal side to the C-terminal side from the amino acid sequence set forth in SEQ ID NO: 6, and further inserting one [$(A)_n$ motif-REP] before the C-terminal sequence. The amino acid sequence set forth in SEQ ID NO: 8 is an amino acid sequence obtained by inserting two alanine residues on the C-terminal side of each $(A)_n$ motif of the amino acid sequence set forth in SEQ ID NO: 7, and further substituting a part of glutamine (Q) residues with a serine (S) residue, and deleting a part of amino acids on the N-terminal side so that the molecular weight thereof is approximately the same as that of SEQ ID NO: 7. The amino acid sequence set forth in SEQ ID NO: 9 is an amino acid sequence obtained by adding a His tag to the C-terminal of the sequence having four repeating regions of 20 domain sequences existing in the amino acid residue set forth in SEQ ID NO: 11 (where several amino acid residues on the C-terminal side of the region are substituted).

The value of z/w in the amino acid sequence set forth in SEQ ID NO: 10 (corresponding to naturally occurring spider fibroin) is 46.8%. The values of z/w of the amino acid sequence set forth in SEQ ID NO: 6, the amino acid sequence set forth in SEQ ID NO: 7, the amino acid sequence set forth in SEQ ID NO: 8, and the amino acid sequence set forth in SEQ ID NO: 9 are respectively 58.7%, 70.1%, 66.1%, and 70.0%. Additionally, the values of x/y at the Giza ratio (described later) of 1:1.8 to 11.3 of the amino acid sequences set forth in SEQ ID NO: 10, SEQ ID NO: 6, SEQ ID NO: 7, SEQ ID NO: 8, and SEQ ID NO: 9 are respectively 15.0%, 15.0%, 93.4%, 92.7%, and 89.3%.

The modified fibroin of (2-i) may consist of the amino acid sequence set forth in SEQ ID NO: 6, SEQ ID NO: 7, SEQ ID NO: 8, or SEQ ID NO: 9.

The modified fibroin of (2-ii) includes an amino acid sequence having a sequence identity of 90% or more with the amino acid sequence set forth in SEQ ID NO: 6, SEQ ID NO: 7, SEQ ID NO: 8, or SEQ ID NO: 9. The modified fibroin of (2-ii) is also a protein including a domain sequence represented by Formula 1: $[(A)_n \text{ motif-REP}]_m$. The sequence identity is preferably 95% or more.

It is preferred that the modified fibroin of (2-ii) has a sequence identity of 90% or more with the amino acid sequence set forth in SEQ ID NO: 6, SEQ ID NO: 7, SEQ ID NO: 8, or SEQ ID NO: 9, and z/w is 50.9% or more in a case where the total number of amino acid residues in the amino acid sequence consisting of XGX (where X represents an amino acid residue other than glycine) included in REP is defined as z, and the total number of amino acid residues of REP in the domain sequence is defined as w.

The second modified fibroin may include a tag sequence at either or both of the N-terminal and the C-terminal. This enables the modified fibroin to be isolated, immobilized, detected and visualized.

The tag sequence may be, for example, an affinity tag utilizing specific affinity (binding property, affinity) with another molecule. A specific example of the affinity tag includes a histidine tag (His tag). The His tag is a short peptide in which about 4 to 10 histidine residues are arranged, and has a property of specifically binding to a metal ion such as nickel, and thus can be used for isolation of modified fibroin by chelating metal chromatography. A specific example of the tag sequence may be, for example, the amino acid sequence set forth in SEQ ID NO: 12 (amino acid sequence including a His tag and a hinge sequence).

Also, a tag sequence such as glutathione-S-transferase (GST) that specifically binds to glutathione, and a maltose binding protein (MBP) that specifically binds to maltose can also be utilized.

Further, an "epitope tag" utilizing an antigen-antibody reaction can also be utilized. Adding a peptide (epitope) exhibiting antigenicity as a tag sequence allows an antibody against the epitope to be bound. Examples of the epitope tag include an HA (peptide sequence of hemagglutinin of influenza virus) tag, a myc tag, and a FLAG tag. The modified fibroin can easily be purified with high specificity by utilizing an epitope tag.

Moreover, it is possible to use a tag sequence which can be cleaved with a specific protease. The modified fibroin cleaved from the tag sequence can be recovered by treating a protein adsorbed through the tag sequence with protease.

More specific examples of the second modified fibroin including a tag sequence include modified fibroins including (2-iii) the amino acid sequence set forth in SEQ ID NO: 13, SEQ ID NO: 11, SEQ ID NO: 14, or SEQ ID NO: 15, or (2-iv) an amino acid sequence having a sequence identity of 90% or more with the amino acid sequence set forth in SEQ ID NO: 13, SEQ ID NO: 11, SEQ ID NO: 14, or SEQ ID NO: 15.

The amino acid sequences set forth in SEQ ID NO: 16, SEQ ID NO: 17, SEQ ID NO: 13, SEQ ID NO: 11, SEQ ID NO: 14, and SEQ ID NO: 15 are an amino acid sequence obtained by adding the amino acid sequence set forth in SEQ ID NO: 12 (including a His tag sequence and a hinge sequence) to the N-terminal of each of the amino acid sequences set forth in SEQ ID NO: 10, SEQ ID NO: 18, SEQ ID NO: 6, SEQ ID NO: 7, SEQ ID NO: 8, and SEQ ID NO: 9.

The modified fibroin of (2-iii) may consist of the amino acid sequence set forth in SEQ ID NO: 13, SEQ ID NO: 11, SEQ ID NO: 14, or SEQ ID NO: 15.

The modified fibroin of (2-iv) includes an amino acid sequence having a sequence identity of 90% or more with the amino acid sequence set forth in SEQ ID NO: 13, SEQ ID NO: 11, SEQ ID NO: 14, or SEQ ID NO: 15. The modified fibroin of (2-iv) is also a protein including a domain sequence represented by Formula 1: $[(A)_n$ motif-REP$]_m$. The sequence identity is preferably 95% or more.

It is preferred that the modified fibroin of (2-iv) has a sequence identity of 90% or more with the amino acid sequence set forth in SEQ ID NO: 13, SEQ ID NO: 11, SEQ ID NO: 14, or SEQ ID NO: 15, and z/w is 50.9% or more in a case where the total number of amino acid residues in the amino acid sequence consisting of XGX (where X represents an amino acid residue other than glycine) contained in REP is defined as z, and the total number of amino acid residues of REP in the domain sequence is defined as w.

The second modified fibroin may include a secretory signal for releasing the protein produced in the recombinant protein production system to the outside of a host. The sequence of the secretory signal can be appropriately set depending on the type of the host.

The modified fibroin having a reduced content of $(A)_n$ motif (third modified fibroin) has a domain sequence having an amino acid sequence having a reduced content of $(A)_n$ motif, as compared to naturally occurring spider fibroin. The domain sequence of the third modified fibroin may have an amino acid sequence corresponding to an amino acid sequence in which at least one or a plurality of $(A)_n$ motifs are deleted, as compared to naturally occurring spider fibroin.

The third modified fibroin may have an amino acid sequence corresponding to an amino acid sequence in which 10 to 40% of the $(A)_n$ motif is deleted from naturally occurring spider fibroin.

The third modified fibroin may have a domain sequence having an amino acid sequence corresponding to an amino acid sequence in which at least one $(A)_n$ motif every one to three $(A)_n$ motifs from the N-terminal side to the C-terminal side is deleted, as compared to naturally occurring spider fibroin.

The third modified fibroin may have a domain sequence having an amino acid sequence corresponding to an amino acid sequence in which at least two consecutive $(A)_n$ motif deletions and one $(A)_n$ motif deletion are repeated in this order from the N-terminal side to the C-terminal side, as compared to naturally occurring spider fibroin.

The third modified fibroin may have a domain sequence having an amino acid sequence corresponding to an amino acid sequence in which at least $(A)_n$ motif every other two positions is deleted from the N-terminal side to the C-terminal side.

The third modified fibroin has a domain sequence represented by Formula 1: $[(A)_n$ motif-REP$]_m$, and may have an amino acid sequence in which x/y is 20% or more, 30% or more, 40% or more, or 50% or more in a case where the number of amino acid residues in REPs of two adjacent $[(A)_n$ motif-REP] units is sequentially compared from the N-terminal side to the C-terminal side, and the number of amino acid residues in REP having a smaller number of amino acid residues is defined as 1, the maximum value of the total value of the number of amino acid residues in the two adjacent $[(A)_n$ motif-REP] units where the ratio of the number of amino acid residues in the other REP is 1.8 to 11.3 is defined as x, and the total number of amino acid residues of the domain sequence is defined as y. The number of alanine residues relative to the total number of amino acid residues in the $(A)_n$ motif may be 83% or more, preferably 86% or more, more preferably 90% or more, still more preferably 95% or more, and even still more preferably 100% (which means that the $(A)_n$ motif consists of only alanine residues).

A method of calculating x/y will be described in more detail with reference to Fig. 1. Fig. 1 shows a domain sequence excluding the N-terminal sequence and the C-terminal sequence from spider fibroin. The domain sequence has a sequence of $(A)_n$ motif-first REP (50 amino acid residues)-$(A)_n$ motif-second REP (100 amino acid residues)-$(A)_n$ motif-third REP (10 amino acid residues)-$(A)_n$ motif-fourth REP (20 amino acid residues)-$(A)_n$ motif-fifth REP (30 amino acid residues)-$(A)_n$ motif from the N-terminal side (left side).

The two adjacent $[(A)_n$ motif-REP] units are sequentially selected from the N-terminal side to the C-terminal side so as not to overlap. At this time, an unselected $[(A)_n$ motif-REP] unit may exist. Fig. 1 shows a pattern 1 (a comparison between the first REP and the second REP, and a comparison between the third REP and the fourth REP), a pattern 2 (a comparison between the first REP and the second REP, and a comparison between the fourth REP and the fifth REP), a pattern 3 (a comparison between the second REP and the third REP, and a comparison between the fourth REP and the fifth REP), and a pattern 4 (a comparison between the first REP and the second REP). There are other selection methods besides this.

Subsequently, the number of amino acid residues of each REP in the selected two adjacent $[(A)_n$ motif-REP] units is compared for each pattern. The comparison is performed by determining the ratio of the number of amino acid residues of the other REP in a case where one REP having a smaller number of amino acid residues is defined as 1. For example, in a case of comparing the first REP (50 amino acid residues) and the second REP (100 amino acid residues), the ratio of the number of amino acid residues of the second REP is 100/50 = 2 in a case where the first REP having a smaller number of amino acid residues is defined as 1. Similarly, in a case of comparing the fourth REP (20 amino acid residues) and the fifth REP (30 amino acid residues), the ratio of the number of amino acid residues of the fifth REP is 30/20 = 1.5 in a case where

the fourth REP having a smaller number of amino acid residues is defined as 1.

In Fig. 1, a set of [$(A)_n$ motif-REP] units in which the ratio of the number of amino acid residues of the other REP is 1.8 to 11.3 in a case where one REP having a smaller number of amino acid residues is defined as 1 is indicated by a solid line. Hereinafter, such a ratio is referred to as a Giza ratio. A set of [$(A)_n$ motif-REP] units in which the ratio of the number of amino acid residues of the other REP is less than 1.8 or more than 11.3 in a case where one REP having a smaller number of amino acid residues is defined as 1 is indicated by a broken line.

In each pattern, the number of all amino acid residues of two adjacent [$(A)_n$ motif-REP] units indicated by solid lines (including not only the number of amino acid residues of the REP but also the number of amino acid residues of the $(A)_n$ motif) is combined. Then, the total values combined are compared, and the total value of the pattern whose total value is the maximum (the maximum value of the total value) is defined as x. In the example shown in Fig. 1, the total value of the pattern 1 is the maximum.

Then, x/y (%) can be calculated by dividing x by the total number of amino acid residues y of the domain sequence.

In the third modified fibroin, x/y is preferably 50% or more, more preferably 60% or more, still more preferably 65% or more, even still more preferably 70% or more, still further preferably 75% or more, and particularly preferably 80% or more. The upper limit of x/y is not particularly limited, but may be 100% or less, for example. In a case where the Giza ratio is 1 : 1.9 to 11.3, x/y is preferably 89.6% or more; in a case where the Giza ratio is 1 : 1.8 to 3.4, x/y is preferably 77.1% or more; in a case where the Giza ratio is 1 : 1.9 to 8.4, x/y is preferably 75.9% or more; and in a case where the Giza ratio is 1 : 1.9 to 4.1, x/y is preferably 64.2% or more.

In a case where the third modified fibroin is a modified fibroin in which at least seven of a plurality of $(A)_n$ motifs in the domain sequence consist of only alanine residues, x/y is preferably 46.4% or more, more preferably 50% or more, still more preferably 55% or more, even still more preferably 60% or more, still further preferably 70% or more, and particularly preferably 80% or more. The upper limit of x/y is not particularly limited, but is only required to be 100% or less.

The third modified fibroin can be obtained by, for example, deleting one or a plurality of sequences encoding the $(A)_n$ motif from the gene sequence of cloned naturally occurring spider fibroin so that x/y is 64.2% or more. Alternatively, the third modified fibroin can also be obtained by, for example, designing an amino acid sequence corresponding to an amino acid sequence in which one or a plurality of $(A)_n$ motifs are deleted from the amino acid sequence of naturally occurring spider fibroin so that x/y is 64.2% or more, and chemically synthesizing a nucleic acid encoding the designed amino acid sequence. In any case, in addition to modification corresponding to deletion of the $(A)_n$ motif from the amino acid sequence of naturally occurring spider fibroin, modification of the amino acid sequence corresponding to substitution, deletion, insertion, and/or addition of one or a plurality of amino acid residues may be further performed.

More specific examples of the third modified fibroin include modified fibroins including (3-i) the amino acid sequence set forth in SEQ ID NO: 18, SEQ ID NO: 7, SEQ ID NO: 8, or SEQ ID NO: 9, or (3-ii) an amino acid sequence having a sequence identity of 90% or more with the amino acid sequence set forth in SEQ ID NO: 18, SEQ ID NO: 7, SEQ ID NO: 8, or SEQ ID NO: 9.

The modified fibroin of (3-i) will be described. The amino acid sequence set forth in SEQ ID NO: 18 is an amino acid sequence obtained by deleting the $(A)_n$ motif every other two positions from the N-terminal side to the C-terminal side from the amino acid sequence set forth in SEQ ID NO: 10 corresponding to naturally occurring spider fibroin, and further inserting one [$(A)_n$ motif-REP] before the C-terminal sequence. The amino acid sequence set forth in SEQ ID NO: 7 is an amino acid sequence obtained by substituting all GGXs in REP of the amino acid sequence set forth in SEQ ID NO: 18 with GQX. The amino acid sequence set forth in SEQ ID NO: 8 is an amino acid sequence obtained by inserting two alanine residues on the C-terminal side of each $(A)_n$ motif of the amino acid sequence set forth in SEQ ID NO: 7, and further substituting a part of glutamine (Q) residues with a serine (S) residue, and deleting a part of amino acids on the N-terminal side so that the molecular weight thereof is approximately the same as that of SEQ ID NO: 7. The amino acid sequence set forth in SEQ ID NO: 9 is an amino acid sequence obtained by adding a His tag to the C-terminal of the sequence having four repeating regions of 20 domain sequences existing in the amino acid residue set forth in SEQ ID NO: 11 (where several amino acid residues on the C-terminal side of the region are substituted).

The value of x/y at the Giza ratio of 1 : 1.8 to 11.3 of the amino acid sequence set forth in SEQ ID NO: 10 (corresponding to naturally occurring spider fibroin) is 15.0%. The values of x/y of the amino acid sequence set forth in SEQ ID NO: 18 and the amino acid sequence set forth in SEQ ID NO: 7 are both 93.4%. The value of x/y of the amino acid sequence set forth in SEQ ID NO: 8 is 92.7%. The value of x/y of the amino acid sequence set forth in SEQ ID NO: 9 is 89.3%. The values of z/w of the amino acid sequences set forth in SEQ ID NO: 10, SEQ ID NO: 18, SEQ ID NO: 7, SEQ ID NO: 8, and SEQ ID NO: 9 are respectively 46.8%, 56.2%, 70.1%, 66.1%, and 70.0%.

The modified fibroin of (3-i) may consist of the amino acid sequence set forth in SEQ ID NO: 18, SEQ ID NO: 7, SEQ ID NO: 8, or SEQ ID NO: 9.

The modified fibroin of (3-ii) includes an amino acid sequence having a sequence identity of 90% or more with the amino acid sequence set forth in SEQ ID NO: 18, SEQ ID NO: 7, SEQ ID NO: 8, or SEQ ID NO: 9. The modified fibroin of (3-ii) is also a protein including a domain sequence represented by Formula 1: [$(A)_n$ motif-REP]$_m$. The sequence identity is

preferably 95% or more.

It is preferred that the modified fibroin of (3-ii) has a sequence identity of 90% or more with the amino acid sequence set forth in SEQ ID NO: 18, SEQ ID NO: 7, SEQ ID NO: 8, or SEQ ID NO: 9, and x/y is 64.2% or more in a case where the number of amino acid residues in REPs of two adjacent $[(A)_n$ motif-REP] units is sequentially compared from the N-terminal side to the C-terminal side, and the number of amino acid residues in REP having a smaller number of amino acid residues is defined as 1, the maximum value of the total value of the number of amino acid residues in the two adjacent $[(A)_n$ motif-REP] units where the ratio of the number of amino acid residues in the other REP is 1.8 to 11.3 (the Giza ratio is 1 : 1.8 to 11.3) is defined as x, and the total number of amino acid residues of the domain sequence is defined as y.

The third modified fibroin may include the above-described tag sequence at either or both of the N-terminal and the C-terminal.

More specific examples of the third modified fibroin including a tag sequence include modified fibroins including (3-iii) the amino acid sequence set forth in SEQ ID NO: 17, SEQ ID NO: 11, SEQ ID NO: 14, or SEQ ID NO: 15, or (3-iv) an amino acid sequence having a sequence identity of 90% or more with the amino acid sequence set forth in SEQ ID NO: 17, SEQ ID NO: 11, SEQ ID NO: 14, or SEQ ID NO: 15.

The amino acid sequences set forth in SEQ ID NO: 16, SEQ ID NO: 17, SEQ ID NO: 13, SEQ ID NO: 11, SEQ ID NO: 14, and SEQ ID NO: 15 are an amino acid sequence obtained by adding the amino acid sequence set forth in SEQ ID NO: 12 (including a His tag sequence and a hinge sequence) to the N-terminal of each of the amino acid sequences set forth in SEQ ID NO: 10, SEQ ID NO: 18, SEQ ID NO: 6, SEQ ID NO: 7, SEQ ID NO: 8, and SEQ ID NO: 9.

The modified fibroin of (3-iii) may consist of the amino acid sequence set forth in SEQ ID NO: 17, SEQ ID NO: 11, SEQ ID NO: 14, or SEQ ID NO: 15.

The modified fibroin of (3-iv) includes an amino acid sequence having a sequence identity of 90% or more with the amino acid sequence set forth in SEQ ID NO: 17, SEQ ID NO: 11, SEQ ID NO: 14, or SEQ ID NO: 15. The modified fibroin of (3-iv) is also a protein including a domain sequence represented by Formula 1: $[(A)_n$ motif-REP$]_m$. The sequence identity is preferably 95% or more.

It is preferred that the modified fibroin of (3-iv) has a sequence identity of 90% or more with the amino acid sequence set forth in SEQ ID NO: 17, SEQ ID NO: 11, SEQ ID NO: 14, or SEQ ID NO: 15, and x/y is 64.2% or more in a case where the number of amino acid residues in REPs of two adjacent $[(A)_n$ motif-REP] units is sequentially compared from the N-terminal side to the C-terminal side, and the number of amino acid residues in REP having a smaller number of amino acid residues is defined as 1, the maximum value of the total value of the number of amino acid residues in the two adjacent $[(A)_n$ motif-REP] units where the ratio of the number of amino acid residues in the other REP is 1.8 to 11.3 is defined as x, and the total number of amino acid residues of the domain sequence is defined as y.

The third modified fibroin may include a secretory signal for releasing the protein produced in the recombinant protein production system to the outside of a host. The sequence of the secretory signal can be appropriately set depending on the type of the host.

The modified fibroin having a reduced content of glycine residue and a reduced content of $(A)_n$ motif (fourth modified fibroin) has a domain sequence having an amino acid sequence having a reduced content of glycine residue, in addition to having a reduced content of $(A)_n$ motif, as compared to naturally occurring spider fibroin. The fourth modified fibroin may have a domain sequence having an amino acid sequence corresponding to an amino acid sequence in which, in addition to deletion of at least one or a plurality of $(A)_n$ motifs, at least one or a plurality of glycine residues in REP are substituted with another amino acid residue, as compared to naturally occurring spider fibroin. That is, the fourth modified fibroin is a modified fibroin having characteristic of both the modified fibroin having a reduced content of glycine residue (second modified fibroin) and the modified fibroin having a reduced content of $(A)_n$ motif (third modified fibroin) described above. Specific embodiments thereof, and the like are as in the descriptions for the second modified fibroin and the third modified fibroin.

More specific examples of the fourth modified fibroin include modified fibroins including (4-i) the amino acid sequence set forth in SEQ ID NO: 7, SEQ ID NO: 8, SEQ ID NO: 9, SEQ ID NO: 11, SEQ ID NO: 14, or SEQ ID NO: 15, or (4-ii) an amino acid sequence having a sequence identity of 90% or more with the amino acid sequence set forth in SEQ ID NO: 7, SEQ ID NO: 8, SEQ ID NO: 9, SEQ ID NO: 11, SEQ ID NO: 14, or SEQ ID NO: 15. Specific embodiments of the modified fibroin including the amino acid sequence set forth in SEQ ID NO: 7, SEQ ID NO: 8, SEQ ID NO: 9, SEQ ID NO: 11, SEQ ID NO: 14, or SEQ ID NO: 15 are as described above.

The modified fibroin including a domain sequence having a region with locally high hydropathy index (fifth modified fibroin) may have a domain sequence having an amino acid sequence including a region with locally high hydropathy index, corresponding to an amino acid sequence in which one or a plurality of amino acid residues in REP are substituted with an amino acid residue with a high hydropathy index and/or one or a plurality of amino acid residues with a high hydropathy index are inserted in REP, as compared to naturally occurring spider fibroin.

The region with locally high hydropathy index preferably consists of consecutive two to four amino acid residues.

The above-described amino acid residue with a high hydropathy index is more preferably an amino acid residue selected from isoleucine (I), valine (V), leucine (L), phenylalanine (F), cysteine (C), methionine (M), and alanine (A).

In the fifth modified fibroin, in addition to modifications corresponding to substitution of one or a plurality of amino acid residues in REP with an amino acid residue with a high hydropathy index and/or insertion of one or a plurality of amino acid residues with a high hydropathy index into REP, as compared to naturally occurring spider fibroin, there may be further modification of an amino acid sequence corresponding to substitution, deletion, insertion, and/or addition of one or a plurality of amino acid residues as compared to naturally occurring spider fibroin.

The fifth modified fibroin can be obtained by, for example, substituting one or a plurality of hydrophilic amino acid residues in REP (for example, amino acid residues having a negative hydropathy index) with a hydrophobic amino acid residue (for example, an amino acid residue having a positive hydropathy index), and/or inserting one or a plurality of hydrophobic amino acid residues into REP, from the gene sequence of cloned naturally occurring spider fibroin. Alternatively, the fifth modified fibroin can be obtained by, for example, designing an amino acid sequence corresponding to an amino acid sequence in which one or a plurality of hydrophilic amino acid residues in REP are substituted with a hydrophobic amino acid residue and/or one or a plurality of hydrophobic amino acid residues are inserted into REP, from the amino acid sequence of naturally occurring spider fibroin, and chemically synthesizing a nucleic acid encoding the designed amino acid sequence. In any case, in addition to modifications corresponding to substitution of one or a plurality of hydrophilic amino acid residues in REP with a hydrophobic amino acid residue, and/or insertion of one or a plurality of hydrophobic amino acid residues into REP, from the amino acid sequence of naturally occurring spider fibroin, modification of the amino acid sequence corresponding to substitution, deletion, insertion, and/or addition of one or a plurality of amino acid residues may be further performed.

The fifth modified fibroin includes a domain sequence represented by Formula 1: $[(A)_n \text{ motif-REP}]_m$, and may have an amino acid sequence in which p/q is 6.2% or more in a case where in all REPs included in a sequence excluding the sequence from the $(A)_n$ motif located at the most C-terminal side to the C-terminal of the domain sequence from the domain sequence, the total number of amino acid residues included in a region where the average value of hydropathy indices of four consecutive amino acid residues is 2.6 or more is defined as p, and the total number of amino acid residues included in a sequence excluding the sequence from the $(A)_n$ motif located at the most C-terminal side to the C-terminal of the domain sequence from the domain sequence is defined as q.

For the hydropathy index of the amino acid residue, a publicly known index (Hydropathy index: Kyte J, & Doolittle R (1982)" A simple method for displaying the hydropathic character of a protein", J. Mol. Biol., 157, pp.105-132) is used. Specifically, the hydropathy index (hereinafter, also referred to as "HI") of each amino acid is as shown in Table 2 below.

[Table 2]

| Amino acid | HI | Amino acid | HI |
|---|---|---|---|
| Isoleucine (Ile) | 4.5 | Tryptophan (Trp) | -0.9 |
| Valine (Val) | 4.2 | Tyrosine (Tyr) | -1.3 |
| Leucine (Leu) | 3.8 | Proline (Pro) | -1.6 |
| Phenylalanine (Phe) | 2.8 | Histidine (His) | -3.2 |
| Cysteine (Cys) | 2.5 | Asparagine (Asn) | -3.5 |
| Methionine (Met) | 1.9 | Aspartic acid (Asp) | -3.5 |
| Alanine (Ala) | 1.8 | Glutamine (Gln) | -3.5 |
| Glycine (Gly) | -0.4 | Glutamine acid (Glu) | -3.5 |
| Threonine (Thr) | -0. 7 | Lysine (Lys) | -3.9 |
| Serine (Ser) | -0.8 | Arginine (Arg) | -4.5 |

The calculation method of p/q will be described in more detail. In the calculation, a sequence excluding the sequence from the $(A)_n$ motif located at the most C-terminal side to the C-terminal of the domain sequence from the domain sequence represented by Formula 1 $[(A)_n \text{ motif-REP}]_m$ (hereinafter also referred to as "sequence A") is used. First, in all REPs included in the sequence A, the average values of hydropathy indices of four consecutive amino acid residues are calculated. The average value of the hydropathy indices is determined by dividing the total sum of HIs of respective amino acid residues included in the four consecutive amino acid residues by 4 (number of amino acid residues). The average value of the hydropathy indices is determined for all of the four consecutive amino acid residues (each of the amino acid residues is used for calculating the average value 1 to 4 times). Then, a region where the average value of the hydropathy indices of the four consecutive amino acid residues is 2.6 or more is specified. Even in a case where a certain amino acid residue corresponds to the "four consecutive amino acid residues having an average value of the hydropathy indices of 2.6 or more" multiple times, the amino acid residue is included as one amino acid residue in the region. The total number of

amino acid residues included in the region is p. Also, the total number of amino acid residues included in the sequence A is q.

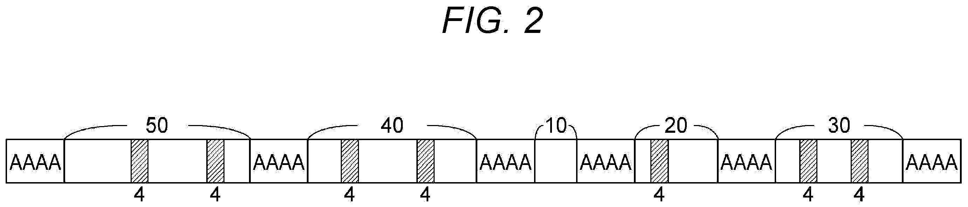

For example, in a case where the "four consecutive amino acid residues having an average value of the hydropathy indices of 2.6 or more" are extracted from 20 places (no overlap), in the region where the average value of the hydropathy indices of four consecutive amino acid residues is 2.6 or more, 20 of the four consecutive amino acid residues (no overlap) are included, and thus p is $20 \times 4 = 80$. Further, for example, in a case where two of the "four consecutive amino acid residues having an average value of the hydropathy indices of 2.6 or more" overlap by one amino acid residue, in the region where the average value of the hydropathy indices of the four consecutive amino acid residues is 2.6 or more, seven amino acid residues are included ($p = 2 \times 4 - 1 = 7$. "-1" is the deduction of overlap). For example, in a case of the domain sequence shown in Fig. 2, there are seven "four consecutive amino acid residues having an average value of the hydropathy indices of 2.6 or more" without overlapping, and thus p is $7 \times 4 = 28$. For example, in a case of the domain sequence shown in Fig. 2, q is 4 + 50 + 4 + 40 + 4 + 10 + 4 + 20 + 4 + 30 = 170 (not including the $(A)_n$ motif located at the end of the C-terminal side). Next, p/q (%) can be calculated by dividing p by q. In a case of Fig. 2, p/q is 28/170 = 16.47%.

In the fifth modified fibroin, p/q is preferably 6.2% or more, more preferably 7% or more, still more preferably 10% or more, even still more preferably 20% or more, and still further preferably 30% or more. The upper limit of p/q is not particularly limited, but may be 45% or less, for example.

The fifth modified fibroin can be obtained by, for example, substituting one or a plurality of hydrophilic amino acid residues in REP (for example, amino acid residues having a negative hydropathy index) with a hydrophobic amino acid residue (for example, an amino acid residue having a positive hydropathy index), and/or inserting one or a plurality of hydrophobic amino acid residues into REP, so as to satisfy the condition of the above p/q, thereby modifying the amino acid sequence of cloned naturally occurring spider fibroin into an amino acid sequence including a region with locally high hydropathy index. Alternatively, the fifth modified fibroin can also be obtained by, for example, designing an amino acid sequence satisfying the condition of the above p/q from the amino acid sequence of naturally occurring spider fibroin, and chemically synthesizing a nucleic acid encoding the designed amino acid sequence. In any case, in addition to modifications corresponding to substitution of one or a plurality of amino acid residues in REP with an amino acid residue with a high hydropathy index, and/or insertion of one or a plurality of amino acid residues with a high hydropathy index into REP, as compared to naturally occurring spider fibroin, modification corresponding to substitution, deletion, insertion, and/or addition of one or a plurality of amino acid residues may be further performed.

The amino acid residue with a high hydropathy index is not particularly limited, but is preferably isoleucine (I), valine (V), leucine (L), phenylalanine (F), cysteine (C), methionine (M), and alanine (A), and more preferably valine (V), leucine (L), and isoleucine (I).

More specific examples of the fifth modified fibroin include modified fibroins including (5-i) the amino acid sequence set forth in SEQ ID NO: 19, SEQ ID NO: 20, or SEQ ID NO: 21, or (5-ii) an amino acid sequence having a sequence identity of 90% or more with the amino acid sequence set forth in SEQ ID NO: 19, SEQ ID NO: 20, or SEQ ID NO: 21.

The modified fibroin of (5-i) will be described. The amino acid sequence set forth in SEQ ID NO: 22 is an amino acid sequence obtained by deleting alanine residues in the $(A)_n$ motif of the amino acid sequence of naturally occurring spider fibroin so that the number of the consecutive alanine residues is five. The amino acid sequence set forth in SEQ ID NO: 19 is an amino acid sequence obtained by inserting an amino acid sequence consisting of three amino acid residues (VLI) at two sites for each REP into the amino acid sequence set forth in SEQ ID NO: 22, and deleting a part of amino acids on the C-terminal side so that the molecular weight thereof is approximately the same as that of the amino acid sequence set forth in SEQ ID NO: 22. The amino acid sequence set forth in SEQ ID NO: 23 is an amino acid sequence obtained by inserting two alanine residues at the C-terminal side of each $(A)_n$ motif of the amino acid sequence set forth in SEQ ID NO: 22, further substituting a part of glutamine (Q) residues with a serine (S) residue, and deleting a part of amino acids on the C-terminal side so that the molecular weight thereof is approximately the same as that of the amino acid sequence set forth in SEQ ID NO: 22. The amino acid sequence set forth in SEQ ID NO: 20 is an amino acid sequence obtained by inserting an amino acid sequence consisting of three amino acid residues (VLI) at one site for each REP into the amino acid sequence set forth in SEQ ID NO: 23. The amino acid sequence set forth in SEQ ID NO: 21 is an amino acid sequence obtained by inserting an amino acid sequence consisting of three amino acid residues (VLI) at two sites for each REP into the amino acid sequence set forth in SEQ ID NO: 23.

The modified fibroin of (5-i) may consist of the amino acid sequence set forth in SEQ ID NO: 19, SEQ ID NO: 20, or SEQ ID NO: 21.

The modified fibroin of (5-ii) includes an amino acid sequence having a sequence identity of 90% or more with the amino acid sequence set forth in SEQ ID NO: 19, SEQ ID NO: 20, or SEQ ID NO: 21. The modified fibroin of (5-ii) is also a protein including a domain sequence represented by Formula 1: $[(A)_n \text{ motif-REP}]_m$. The sequence identity is preferably 95% or more.

It is preferred that the modified fibroin of (5-ii) has a sequence identity of 90% or more with the amino acid sequence set forth in SEQ ID NO: 19, SEQ ID NO: 20, or SEQ ID NO: 21, and p/q is 6.2% or more in a case where in all

REPs included in a sequence excluding the sequence from the $(A)_n$ motif located at the most C-terminal side to the C-terminal of the domain sequence from the domain sequence, the total number of amino acid residues included in a region where the average value of hydropathy indices of four consecutive amino acid residues is 2.6 or more is defined as p, and the total number of amino acid residues included in a sequence excluding the sequence from the $(A)_n$ motif located at the most the C-terminal side to the C-terminal of the domain sequence from the domain sequence is defined as q.

The fifth modified fibroin may include a tag sequence at either or both of the N-terminal and the C-terminal.

More specific examples of the fifth modified fibroin including a tag sequence include modified fibroins including (5-iii) the amino acid sequence set forth in SEQ ID NO: 24, SEQ ID NO: 25, or SEQ ID NO: 26, or (5-iv) an amino acid sequence having a sequence identity of 90% or more with the amino acid sequence set forth in SEQ ID NO: 24, SEQ ID NO: 25, or SEQ ID NO: 26.

The amino acid sequences set forth in SEQ ID NO: 24, SEQ ID NO: 25, and SEQ ID NO: 26 are an amino acid sequence obtained by adding the amino acid sequence set forth in SEQ ID NO: 12 (including a His tag sequence and a hinge sequence) to the N-terminal of each of the amino acid sequences set forth in SEQ ID NO: 19, SEQ ID NO: 20, and SEQ ID NO: 21.

The modified fibroin of (5-iii) may consist of the amino acid sequence set forth in SEQ ID NO: 24, SEQ ID NO: 25, or SEQ ID NO: 26.

The modified fibroin of (5-iv) includes an amino acid sequence having a sequence identity of 90% or more with the amino acid sequence set forth in SEQ ID NO: 24, SEQ ID NO: 25, or SEQ ID NO: 26. The modified fibroin of (5-iv) is also a protein including a domain sequence represented by Formula 1: $[(A)_n \text{ motif-REP}]_m$. The sequence identity is preferably 95% or more.

It is preferred that the modified fibroin of (5-iv) has a sequence identity of 90% or more with the amino acid sequence set forth in SEQ ID NO: 24, SEQ ID NO: 25, or SEQ ID NO: 26, and p/q is 6.2% or more in a case where in all REPs included in a sequence excluding the sequence from the $(A)_n$ motif located at the most C-terminal side to the C-terminal of the domain sequence from the domain sequence, the total number of amino acid residues included in a region where the average value of hydropathy indices of four consecutive amino acid residues is 2.6 or more is defined as p, and the total number of amino acid residues included in a sequence excluding the sequence from the $(A)_n$ motif located at the most the C-terminal side to the C-terminal of the domain sequence from the domain sequence is defined as q.

The fifth modified fibroin may include a secretory signal for releasing the protein produced in the recombinant protein production system to the outside of a host. The sequence of the secretory signal can be appropriately set depending on the type of the host.

The modified fibroin, which has a domain sequence having a reduced content of glutamine residue (sixth modified fibroin), has an amino acid sequence having a reduced content of glutamine residue, as compared to naturally occurring spider fibroin.

In the sixth modified fibroin, at least one motif selected from a GGX motif and a GPGXX motif is preferably included in the amino acid sequence of REP.

In a case where the sixth modified fibroin includes the GPGXX motif in REP, the GPGXX motif content is usually 1% or more, may also be 5% or more, and preferably 10% or more. The upper limit of the GPGXX motif content is not particularly limited, and may be 50% or less, or may be 30% or less.

In the present specification, the "GPGXX motif content" is a value calculated by the following method.

The GPGXX motif content is calculated as s/t in a case where in all REPs included in a sequence excluding the sequence from the $(A)_n$ motif located at the most C-terminal side to the C-terminal of the domain sequence in spider fibroin including a domain sequence represented by Formula 1: $[(A)_n \text{ motif-REP}]_m$ or Formula 2: $[(A)_n \text{ motif -REP}]_m\text{-}(A)_n$ motif, a number three times the total number of GPGXX motifs included in this region is defined as s (that is, a number corresponding to the total number of G and P in the GPGXX motif), and the total number of amino acid residues in all REPs excluding the sequence excluding the sequence from the $(A)_n$ motif located at the most C-terminal side to the C-terminal of the domain sequence from the domain sequence and further excluding the $(A)_n$ motifs is defined as t.

In the calculation of the GPGXX motif content, the "sequence excluding the sequence from the $(A)_n$ motif located at the most C-terminal side to the C-terminal of the domain sequence from the domain sequence" is used as a target. In "the sequence from the $(A)_n$ motif located at the most C-terminal side to the C-terminal of the domain sequence" (the sequence corresponding to REP), a sequence having low correlation with a sequence characteristic of spider fibroin is included in some cases, and in a case where m is small, (that is, the domain sequence is short), such a sequence affects the calculation result of the GPGXX motif content. The reason for targeting the sequence is for eliminating this influence. Incidentally, in a case where the "GPGXX motif" is located at the C-terminal of REP, even when "XX" is "AA", for example, it is treated as the "GPGXX motif".

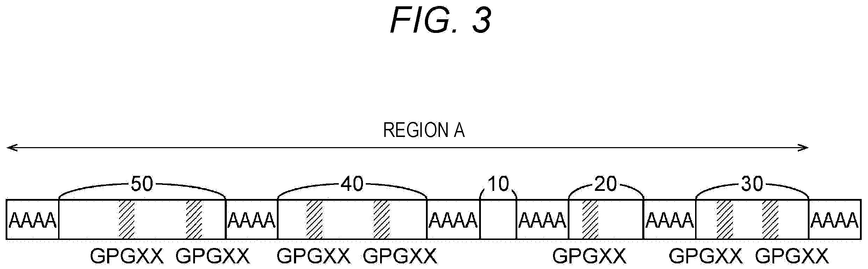

Fig. 3 is a schematic view illustrating the domain sequence of spider fibroin. The calculation method of the GPGXX motif content will be specifically described with reference to Fig. 3. First, in the domain sequence of the spider fibroin shown in Fig. 3 ("$[(A)_n \text{ motif-REP}]_m\text{-}(A)_n$ motif" type), all REPs are included in the "sequence excluding the sequence from the $(A)_n$ motif located at the most C-terminal side to the C-terminal of the domain sequence from the domain

sequence" (the sequence indicated by the "region A" in Fig. 3). Thus, the number of GPGXX motifs for calculating s is 7, and s is $7 \times 3 = 21$. Similarly, all REPs are included in the sequence excluding the sequence from the $(A)_n$ motif located at the most C-terminal side to the C-terminal of the domain sequence from the domain sequence" (the sequence indicated by the "region A" in Fig. 3). Thus, the total number t of amino acid residues in all REPs further excluding the $(A)_n$ motifs from the sequence is 50 + 40 + 10 + 20 + 30 = 150. Then, s/t (%) can be calculated by dividing s by t, and in a case of the fibroin in Fig. 3, s/t is 21/150 = 14.0%.

In the sixth modified fibroin, the glutamine residue content is preferably 9% or less, more preferably 7% or less, still more preferably 4% or less, and particularly preferably 0%.

In the present specification, the "glutamine residue content" is a value calculated by the following method.

The glutamine residue content is calculated as u/t in a case where in all REPs included in a sequence excluding the sequence from the $(A)_n$ motif located at the most C-terminal side to the C-terminal of the domain sequence from the domain sequence (the sequence correspond to the "region A" in Fig. 3) in spider fibroin including a domain sequence represented by Formula 1: $[(A)_n$ motif-REP$]_m$ or Formula 2: $[(A)_n$ motif -REP$]_m$-$(A)_n$ motif, the total number of glutamine residues included in this region is defined as u, and the total number of amino acid residues in all REPs excluding the sequence from the $(A)_n$ motif located at the most C-terminal side to the C-terminal of the domain sequence from the domain sequence and further excluding the $(A)_n$ motifs is defined as t. In the calculation of the glutamine residue content, the reason for targeting the "sequence excluding the sequence from the $(A)_n$ motif located at the most C-terminal side to the C-terminal of the domain sequence from the domain sequence" is the same as the reason descried above.

The sixth modified fibroin may have a domain sequence having an amino acid sequence corresponding to an amino acid sequence in which one or a plurality of glutamine residues in REP are deleted, or substituted with another amino acid residue, as compared to naturally occurring spider fibroin.

The "another amino acid residue" may be an amino acid residue other than the glutamine residue, but is preferably an amino acid residue with a higher hydropathy index than that of the glutamine residue. The hydropathy index of the amino acid residue is as shown in Table 2.

As shown in Table 2, examples of the amino acid residue with a higher hydropathy index than that of the glutamine residue include amino acid residues selected from isoleucine (I), valine (V), leucine (L), phenylalanine (F), cysteine (C), methionine (M), alanine (A), glycine (G), threonine (T), serine (S), tryptophan (W), tyrosine (Y), proline (P), and histidine (H). Among them, the amino acid residue is more preferably an amino acid residue selected from isoleucine (I), valine (V), leucine (L), phenylalanine (F), cysteine (C), methionine (M), and alanine (A), and still more preferably an amino acid residue selected from isoleucine (I), valine (V), leucine (L), and phenylalanine (F).

In the sixth modified fibroin, the hydrophobicity of REP is preferably -0.8 or more, more preferably -0.7 or more, still more preferably 0 or more, even still more preferably 0.3 or more, and particularly preferably 0.4 or more. The upper limit of REP is not particularly limited, and may be 1.0 or less, or may be 0.7 or less.

In the present specification, the "hydrophobicity of REP" is a value calculated by the following method.

The hydrophobicity of REP is calculated as v/t in a case where in all REPs included in a sequence excluding the sequence from the $(A)_n$ motif located at the most C-terminal side to the C-terminal of the domain sequence from the domain sequence (the sequence corresponding to the "region A" in Fig. 3) in spider fibroin including the domain sequence represented by Formula 1: $[(A)_n$ motif-REP$]_m$ or Formula 2: $[(A)_n$ motif -REP$]_m$-$(A)_n$ motif, the total sum of the hydropathy indices of each of amino acid residues in this region is defined as v, and the total number of amino acid residues in all REPs excluding the sequence from the $(A)_n$ motif located at the most C-terminal side to the C-terminal of the domain sequence from the domain sequence and further excluding the $(A)_n$ motifs is defined as t. In the calculation of the hydrophobicity of REP, the reason for targeting the "sequence excluding the sequence from the $(A)_n$ motif located at the most C-terminal side to the C-terminal of the domain sequence from the domain sequence" is the same as the reason descried above.

In the domain sequence of the sixth modified fibroin, in addition to modifications corresponding to deletion of one or a plurality of glutamine residues in REP and/or substitution of one or a plurality of glutamine residues in REP with another amino acid residue, as compared to naturally occurring spider fibroin, there may be further modification of the amino acid sequence corresponding to substitution, deletion, insertion, and/or addition of one or a plurality of amino acid residues.

The sixth modified fibroin can be obtained by, for example, deleting one or a plurality of glutamine residues in REP and/or substituting one or a plurality of glutamine residues in REP with another amino acid residue, from the gene sequence of cloned naturally occurring spider fibroin. Alternatively, the sixth modified fibroin can be obtained by, for example, designing an amino acid sequence corresponding to an amino acid sequence in which one or a plurality of glutamine residues in REP are deleted and/or one or a plurality of glutamine residues in REP are substituted with another amino acid residue, from the amino acid sequence of naturally occurring spider fibroin, and chemically synthesizing a nucleic acid encoding the designed amino acid sequence.

More specific examples of the sixth modified fibroin include modified fibroins including (6-i) the amino acid sequence set forth in SEQ ID NO: 27, SEQ ID NO: 28, SEQ ID NO: 29, SEQ ID NO: 30, SEQ ID NO: 31, SEQ ID NO: 32, SEQ ID NO: 33, or SEQ ID NO: 43, or (6-ii) an amino acid sequence having a sequence identity of 90% or more with the amino acid sequence set forth in SEQ ID NO: 27, SEQ ID NO: 28, SEQ ID NO: 29, SEQ ID NO: 30, SEQ ID NO: 31, SEQ ID

NO: 32, SEQ ID NO: 33, or SEQ ID NO: 43.

The modified fibroin of (6-i) will be described.

The amino acid sequence set forth in SEQ ID NO: 7 (Met-PRT410) is an amino acid sequence obtained by, performing modification of amino acid for improving the productivity, such as modification in which an amino acid sequence having consecutive alanine residues in the $(A)_n$ motif is modified so that the number of the consecutive alanine residues becomes five, based on the base sequence and amino acid sequence of *Nephila clavipes* (GenBank Accession No.: P46804.1, GI: 1174415) which is naturally occurring fibroin. Meanwhile, modification for the glutamine residue (Q) is not performed in Met-PRT410, and thus the glutamine residue content thereof is approximately the same as the glutamine residue content of naturally occurring fibroin.

The amino acid sequence set forth in SEQ ID NO: 27 (M_PRT888) is an amino acid sequence obtained by substituting all QQs in Met-PRT410 (SEQ ID NO: 7) with VL.

The amino acid sequence set forth in SEQ ID NO: 28 (M_PRT965) is an amino acid sequence obtained by substituting all QQs in Met-PRT410 (SEQ ID NO: 7) with TS, and substituting the remaining Q with A.

The amino acid sequence set forth in SEQ ID NO: 29 (M_PRT889) is an amino acid sequence obtained by substituting all QQs in Met-PRT410 (SEQ ID NO: 7) with VL, and substituting the remaining Q with I.

The amino acid sequence set forth in SEQ ID NO: 30 (M_PRT916) is an amino acid sequence obtained by substituting all QQs in Met-PRT410 (SEQ ID NO: 7) with VI, and substituting the remaining Q with L.

The amino acid sequence set forth in SEQ ID NO: 31 (M_PRT918) is an amino acid sequence obtained by substituting all QQs in Met-PRT410 (SEQ ID NO: 7) with VF, and substituting the remaining Q with I.

The amino acid sequence set forth in SEQ ID NO: 34 (M_PRT525) is an amino acid sequence obtained by, with respect to Met-PRT410 (SEQ ID NO: 7), inserting two alanine residues into a region $(A_5)$ having consecutive alanine residues, deleting two domain sequences on the C-terminal side so that the molecular weight thereof is approximately the same as that of Met-PRT410, and substituting glutamine residues (Q) at 13 sites with a serine residue (S) or a proline residue (P).

The amino acid sequence set forth in SEQ ID NO: 32 (M_PRT699) is an amino acid sequence obtained by substituting all QQs in M_PRT525 (SEQ ID NO: 34) with VL.

The amino acid sequence set forth in SEQ ID NO: 33 (M_PRT698) is an amino acid sequence obtained by substituting all QQs in M_PRT525 (SEQ ID NO: 34) with VL, and substituting the remaining Q with I.

The amino acid sequence set forth in SEQ ID NO: 43 (Met-PRT966) is an amino acid sequence obtained by substituting all QQs in the amino acid sequence set forth in SEQ ID NO: 9 (amino acid sequence before the amino acid sequence set forth in SEQ ID NO: 42 is added to the C-terminal thereof) with VF, and substituting the remaining Q with I.

In all the amino acid sequences set forth in SEQ ID NO: 27, SEQ ID NO: 28, SEQ ID NO: 29, SEQ ID NO: 30, SEQ ID NO: 31, SEQ ID NO: 32, SEQ ID NO: 33, and SEQ ID NO: 43, the glutamine residue content is 9% or less (Table 3).

[Table 3]

| Modified Fibroin | Glutamine residue content | GPGXX motif content | Hydrophobicity of REP |
|---|---|---|---|
| Met-PRT410 (SEQ ID NO: 7) | 17.7% | 27.9% | -1.52 |
| M-PRT888 (SEQ ID NO: 27) | 6.3% | 27.9% | -0.07 |
| M-PRT965 (SEQ ID NO: 28) | 0.0% | 27.9% | -0.65 |
| M-PRT889 (SEQ ID NO: 29) | 0.0% | 27.9% | 0.35 |
| M-PRT916 (SEQ ID NO: 30) | 0.0% | 27.9% | 0.47 |
| M-PRT918 (SEQ ID NO: 31) | 0.0% | 27.9% | 0.45 |
| M-PRT525 (SEQ ID NO: 34) | 13.7% | 26.4% | -1.24 |
| M-PRT699 (SEQ ID NO: 32) | 3.6% | 26.4% | -0.78 |
| M-PRT698 (SEQ ID NO: 33) | 0.0% | 26.4% | -0.03 |
| Met-PRT966 (SEQ ID NO: 43) | 0.0% | 28.0% | 0.35 |

The modified fibroin of (6-i) may consist of the amino acid sequence set forth in SEQ ID NO: 27, SEQ ID NO: 28, SEQ ID NO: 29, SEQ ID NO: 30, SEQ ID NO: 31, SEQ ID NO: 32, SEQ ID NO: 33, or SEQ ID NO: 43.

The modified fibroin of (6-ii) includes an amino acid sequence having a sequence identity of 90% or more with the amino acid sequence set forth in SEQ ID NO: 27, SEQ ID NO: 28, SEQ ID NO: 29, SEQ ID NO: 30, SEQ ID NO: 31, SEQ ID NO: 32, SEQ ID NO: 33, or SEQ ID NO: 43. The modified fibroin of (6-ii) is also a protein including a domain sequence represented by Formula 1: $[(A)_n \text{ motif-REP}]_m$ or Formula 2: $[(A)_n \text{ motif-REP}]_m\text{-}(A)_n$ motif. The sequence identity is

preferably 95% or more.

In the modified fibroin of (6-ii), the glutamine residue content is preferably 9% or less. In the modified fibroin of (6-ii), the GPGXX motif content is preferably 10% or more.

The sixth modified fibroin may include a tag sequence at either or both of the N-terminal and the C-terminal. This enables the modified fibroin to be isolated, immobilized, detected and visualized.

More specific examples of the sixth modified fibroin including a tag sequence include modified fibroins including (6-iii) the amino acid sequence set forth in SEQ ID NO: 35, SEQ ID NO: 36, SEQ ID NO: 37, SEQ ID NO: 38, SEQ ID NO: 39, SEQ ID NO: 40, SEQ ID NO: 41, or SEQ ID NO: 44, or (6-iv) an amino acid sequence having a sequence identity of 90% or more with the amino acid sequence set forth in SEQ ID NO: 35, SEQ ID NO: 36, SEQ ID NO: 37, SEQ ID NO: 38, SEQ ID NO: 39, SEQ ID NO: 40, SEQ ID NO: 41, or SEQ ID NO: 44.

The amino acid sequences set forth in SEQ ID NO: 35, SEQ ID NO: 36, SEQ ID NO: 37, SEQ ID NO: 38, SEQ ID NO: 39, SEQ ID NO: 40, SEQ ID NO: 41, and SEQ ID NO: 44 are an amino acid sequence obtained by adding the amino acid sequence set forth in SEQ ID NO: 12 (including a His tag sequence and a hinge sequence) to the N-terminal of each of the amino acid sequences set forth in SEQ ID NO: 27, SEQ ID NO: 28, SEQ ID NO: 29, SEQ ID NO: 30, SEQ ID NO: 31, SEQ ID NO: 32, SEQ ID NO: 33, and SEQ ID NO: 43. Since these amino acid sequences are obtained by only adding a tag sequence at the N-terminal, the glutamine residue content does not change, and in all the amino acid sequences set forth in SEQ ID NO: 35, SEQ ID NO: 36, SEQ ID NO: 37, SEQ ID NO: 38, SEQ ID NO: 39, SEQ ID NO: 40, SEQ ID NO: 41, and SEQ ID NO: 44, the glutamine residue content is 9% or less (Table 4).

[Table 4]

| Modified Fibroin | Glutamine residue content | GPGXX motif content | Hydrophobicity of REP |
|---|---|---|---|
| PRT888 (SEQ ID NO: 35) | 6.3% | 27.9% | -0.07 |
| PRT965 (SEQ ID NO: 36) | 0.0% | 27.9% | -0.65 |
| PRT889 (SEQ ID NO: 37) | 0.0% | 27.9% | 0.35 |
| PRT916 (SEQ ID NO: 38) | 0.0% | 27.9% | 0.47 |
| PRT918 (SEQ ID NO: 39) | 0.0% | 27.9% | 0.45 |
| PRT699 (SEQ ID NO: 40) | 3.6% | 26.4% | -0.78 |
| PRT698 (SEQ ID NO: 41) | 0.0% | 26.4% | -0.03 |
| PRT966 (SEQ ID NO: 44) | 0.0% | 28.0% | 0.35 |

The modified fibroin of (6-iii) may consist of the amino acid sequence set forth in SEQ ID NO: 35, SEQ ID NO: 36, SEQ ID NO: 37, SEQ ID NO: 38, SEQ ID NO: 39, SEQ ID NO: 40, SEQ ID NO: 41, or SEQ ID NO: 44.

The modified fibroin of (6-iv) includes an amino acid sequence having a sequence identity of 90% or more with the amino acid sequence set forth in SEQ ID NO: 35, SEQ ID NO: 36, SEQ ID NO: 37, SEQ ID NO: 38, SEQ ID NO: 39, SEQ ID NO: 40, SEQ ID NO: 41, or SEQ ID NO: 44. The modified fibroin of (6-iv) is also a protein including a domain sequence represented by Formula 1: $[(A)_n \text{ motif-REP}]_m$ or Formula 2: $[(A)_n \text{ motif-REP}]_m\text{-}(A)_n$ motif. The sequence identity is preferably 95% or more.

In the modified fibroin of (6-iv), the glutamine residue content is preferably 9% or less. In the modified fibroin of (6-iv), the GPGXX motif content is preferably 10% or more.

The sixth modified fibroin may include a secretory signal for releasing the protein produced in the recombinant protein production system to the outside of a host. The sequence of the secretory signal can be appropriately set depending on the type of the host.

The modified fibroin may also be a modified fibroin having at least two or more characteristics among the characteristics of the first modified fibroin, the second modified fibroin, the third modified fibroin, the fourth modified fibroin, the fifth modified fibroin, and the sixth modified fibroin.

The spider silk protein may be a hydrophilic spider silk protein or a hydrophobic spider silk protein. The hydrophobic spider silk protein is preferably a spider silk protein in which a value (average HI) obtained by determining the total sum of hydropathy indices (HI) of all amino acid residues constituting spider silk protein and then dividing the sum by the total number of amino acid residues is more than -0.8. The hydrophobic spider silk protein has preferably an average HI of -0.6 or more, more preferably -0.4 or more, still more preferably -0.2 or more, and particularly preferably 0 or more. The hydropathy index is as shown in Table 2. The hydrophilic spider silk protein is a spider silk protein having the above average HI of -0.8 or less. The average hydropathy index (HI) of the protein according to the present embodiment is preferably more than -0.8, preferably -0.7 or more, preferably -0.6 or more, more preferably -0.5 or more, preferably -0.4 or more, preferably -0.3 or more, preferably -0.2 or more, preferably -0.1 or more, more preferably 0 or more, more preferably

0.1 or more, more preferably 0.2 or more, still more preferably 0.3 or more, and particularly preferably 0.4 or more.

The HI of each of the amino acid sequences set forth in SEQ ID NO: 11, SEQ ID NO: 15, SEQ ID NO: 35, SEQ ID NO: 36, SEQ ID NO: 37, SEQ ID NO: 38, SEQ ID NO: 39, SEQ ID NO: 40, and SEQ ID NO: 41 is as shown in Table 5. In the calculation of the HI of each amino acid sequence, calculation was performed excluding a sequence having no relationship with the modified fibroin (that is, a sequence corresponding to the amino acid sequence set forth in SEQ ID NO: 12).

[Table 5]

| Amino acid sequence | Hydrophobicity |
|---|---|
| Amino acid sequence set forth in SEQ ID NO: 11 | -0.8 |
| Amino acid sequence set forth in SEQ ID NO: 15 | -0.8 |
| Amino acid sequence set forth in SEQ ID NO: 35 | 0.07 |
| Amino acid sequence set forth in SEQ ID NO: 36 | -0.16 |
| Amino acid sequence set forth in SEQ ID NO: 37 | 0.55 |
| Amino acid sequence set forth in SEQ ID NO: 38 | 0.54 |
| Amino acid sequence set forth in SEQ ID NO: 39 | 0.49 |
| Amino acid sequence set forth in SEQ ID NO: 40 | 0.21 |
| Amino acid sequence set forth in SEQ ID NO: 41 | 0.48 |
| Amino acid sequence set forth in SEQ ID NO: 45 | -0.74 |
| Amino acid sequence set forth in SEQ ID NO: 47 | -1.2 |
| Amino acid sequence set forth in SEQ ID NO: 48 | 0.47 |
| Amino acid sequence set forth in SEQ ID NO: 49 | -0.531 |

Examples of the hydrophobic spider silk protein include the sequence of the first modified fibroin, the sequence of the second modified fibroin, the sequence of the third modified fibroin, the sequence of the fifth modified fibroin, and the sequence of the sixth modified fibroin. More specific examples of the hydrophobic spider silk protein include spider silk proteins including the amino acid sequence set forth in SEQ ID NO: 27, SEQ ID NO: 28, SEQ ID NO: 29, SEQ ID NO: 30, SEQ ID NO: 31, SEQ ID NO: 32, SEQ ID NO: 33, or SEQ ID NO: 43, or the amino acid sequence set forth in SEQ ID NO: 35, SEQ ID NO: 37, SEQ ID NO: 38, SEQ ID NO: 39, SEQ ID NO: 40, SEQ ID NO: 41, or SEQ ID NO: 44.

The hydrophilic spider silk protein may be, for example, the sequence of the fourth modified fibroin described above. More specific examples of the hydrophilic spider silk protein include spider silk proteins including the amino acid sequence set forth in SEQ ID NO: 4, the amino acid sequence set forth in SEQ ID NO: 6, SEQ ID NO: 7, SEQ ID NO: 8, or SEQ ID NO: 9, the amino acid sequence set forth in SEQ ID NO: 13, SEQ ID NO: 11, SEQ ID NO: 14, or SEQ ID NO: 15, the amino acid sequence set forth in SEQ ID NO: 18, SEQ ID NO: 7, SEQ ID NO: 8, or SEQ ID NO: 9, the amino acid sequence set forth in SEQ ID NO: 17, SEQ ID NO: 11, SEQ ID NO: 14, or SEQ ID NO: 15, the amino acid sequence set forth in SEQ ID NO: 19, SEQ ID NO: 20, SEQ ID NO: 21, or SEQ ID NO: 47.

One type of the above-descried spider silk protein can be used alone, or two or more types thereof can be used in combination.

The spider silk protein can be produced by, for example, by using a host transformed with an expression vector having a nucleic acid sequence encoding the spider silk protein and one or a plurality of regulatory sequences operably linked to the nucleic acid sequence to express the nucleic acid.

A method for producing a nucleic acid encoding the spider silk protein is not particularly limited. The nucleic acid can be produced by, for example, a method of performing cloning through amplification of gene encoding natural spider silk protein by polymerase chain reaction (PCR), or a method of producing the nucleic acid by chemical synthesis. The method of chemically synthesizing nucleic acid is not particularly limited, and gene can be chemically synthesized by, for example, based on amino acid sequence information of the spider silk protein obtained from the NCBI web data base, according to a method of linking oligonucleotides automatically synthesized by AKTA oligopilot plus 10/100 (GE Healthcare, Japan), and the like by PCR, for example. At this time, in order to facilitate purification and/or confirmation of the spider silk protein, a nucleic acid encoding spider silk protein consisting of an amino acid sequence in which an amino acid sequence consisting of a start codon and a His 10-tag are added to the N-terminal may be synthesized.

The regulatory sequence is a sequence that controls the expression of a recombinant protein in a host (for example, a promoter, an enhancer, a ribosome binding sequence, and a transcription termination sequence), and can be appropriately selected depending on the type of the host. As the promoter, an inducible promoter that functions in a host

cell, and can induce the expression of a desired spider silk protein may be used. The inducible promoter is a promoter that can control transcription by presence of an inducer (expression inducer), absence of a repressor molecule, or physical factors such as increase or decrease in the temperature, osmotic pressure, pH value, or the like.

The type of the expression vector such as a plasmid vector, a viral vector, a cosmid vector, a fosmid vector, or an artificial chromosome vector can be appropriately selected depending on the type of the host. As the expression vector, an expression vector that can autonomously replicate in a host cell or can be incorporated into a chromosome of a host, and contains a promoter at a position capable of transcribing a nucleic acid encoding spider silk protein is suitably used.

Both prokaryotes, and eukaryotes such as yeast, filamentous fungi, insect cells, animal cells, and plant cells can be suitably used as the host.

In a case where a prokaryote such as bacteria is used as the host, the expression vector is preferably an expression vector that can autonomously replicate in the prokaryote, and contains a promoter, a ribosome binding sequence, a nucleic acid encoding spider silk protein, and a transcription termination sequence. The expression vector may contain gene that controls the promoter.

Examples of the prokaryote include microorganisms belonging to the genus *Escherichia, Brevibacillus, Serratia, Bacillus, Microbacterium, Brevibacterium, Corynebacterium,* and *Pseudomonas*. Examples of the microorganism belonging to the genus *Escherichia* include *Echerichia coli,* and the like. Examples of the microorganism belonging to the genus *Brevibacillus* include *Brevibacillus agri,* and the like. Examples of the microorganism belonging to the genus *Serratia* include *Serratia liquefacience,* and the like. Examples of the microorganism belonging to the genus *Bacillus* include *Bacillus subtilis,* and the like. Examples of the microorganism belonging to the genus *Microbacterium* include *Microbacterium ammoniaphilum,* and the like. Examples of the microorganism belonging to the genus *Brevibacterium* include *Brevibacterium divaricatum,* and the like. Examples of the microorganism belonging to the genus *Corynebacterium* include *Corynebacterium ammoniagenes,* and the like. Examples of the microorganism belonging to the genus *Pseudomonas* include *Pseudomonas putida,* and the like.

In a case where a prokaryote is used as the host, examples of a vector into which a nucleic acid encoding spider silk protein is introduced include pBTrp2 (manufactured by Boehringer Ingelheim GmbH), pGEX (manufactured by Pharmacia), pUC18, pBluescriptII, pSupex, pET22b, pCold, pUB110, and pNCO2 (JP 2002-238569 A).

Examples of the eukaryotic host include yeasts, and filamentous fungi (mold and the like). Examples of the yeast include yeasts belonging to the genus *Saccharomyces, Pichia,* and *Schizosaccharomyces*. Examples of the filamentous fungi include fungi belonging to the genus *Aspergillus, Penicillium,* and *Trichoderma*.

In a case where a eukaryote is used as the host, examples of a vector into which a nucleic acid encoding spider silk protein is introduced include YEp13 (ATCC37115), and YEp24 (ATCC37051). As a method for introducing an expression vector into the host cell, any method can be used as long as it introduces DNA into the host cell. Examples thereof include a method using calcium ions [Proc. Natl. Acad. Sci. USA, 69, 2110 (1972)], an electroporation method, a spheroplast method, a protoplast method, a lithium acetate method, and a competent method.

As a method for expressing a nucleic acid in a host transformed with an expression vector, secretory production, fusion protein expression, and the like, in addition to direct expression, can be performed according to the method described in Molecular Cloning, 2nd edition.

The spider silk protein can be produced by, for example, culturing a transformed host in a culture medium, producing and accumulating spider silk protein in the culture medium, and then collecting the spider silk protein from the culture medium. The method for culturing a transformed host in a culture medium can be performed according to a method commonly used for culturing a host.

In a case where the host is a prokaryote such as *Escherichia coli* or a eukaryote such as yeast, any of a natural medium and a synthetic medium may be used as a culture medium as long as it contains a carbon source, a nitrogen source, an inorganic salt, or the like which can be assimilated by the host and it enables the host to be efficiently cultured.

Any carbon source that can be assimilated by the host may be used as the carbon source. Examples thereof include carbohydrates such as glucose, fructose, sucrose, and molasses, starch, and starch hydrolyzates containing them, organic acids such as acetic acid and propionic acid, and alcohols such as ethanol and propanol.

Examples of the nitrogen source that can be used include ammonium salts of inorganic or organic acids such as ammonia, ammonium chloride, ammonium sulfate, ammonium acetate, and ammonium phosphate, other nitrogen-containing compounds, peptone, meat extract, yeast extract, corn steep liquor, casein hydrolyzate, soybean cake, and soybean cake hydrolyzate, various fermented bacterial cells, and digested products thereof.

Examples of the inorganic salt that can be used include monopotassium phosphate, dipotassium phosphate, magnesium phosphate, magnesium sulfate, sodium chloride, ferrous sulfate, manganese sulfate, copper sulfate, and calcium carbonate.

Prokaryotes such as *Escherichia coli* or eukaryotes such as yeast can be cultured, for example, under aerobic conditions such as shaking culture or aeration agitation submerged culture. The culture temperature is, for example, 15 to 40°C. The culture time is usually 16 hours to 7 days. The pH of the culture medium during culture is preferably maintained at 3.0 to 9.0. The pH of the culture medium can be adjusted by using an inorganic acid, an organic acid, an alkali solution,

urea, calcium carbonate, ammonia, or the like.

Moreover, antibiotics such as ampicillin and tetracycline may be added to the culture medium during culture as necessary. In a case of culturing a microorganism transformed with an expression vector using an inducible promoter as a promoter, an inducer may be added to the culture medium as necessary. For example, in a case of culturing a microorganism transformed with an expression vector using a lac promoter, isopropyl-β-D-thiogalactopyranoside or the like may be added to the medium, and in a case of culturing a microorganism transformed with an expression vector using a trp promoter, indole acrylic acid or the like may be added to the culture medium.

The spider silk protein produced by a transformed host can be isolated and purified by a method commonly used for protein isolation and purification. For example, in a case where the spider silk protein is expressed in a dissolved state in cells, the host cells are recovered by centrifugation after completion of culture, suspended in an aqueous buffer solution, and then disrupted using an ultrasonicator, a French press, a Manton-Gaulin homogenizer, a Dyno-Mill, or the like to obtain a cell-free extract. A purified preparation can be obtained from the supernatant obtained by centrifuging the cell-free extract, according to a method commonly used for protein isolation and purification, that is, a solvent extraction method, a salting-out method using ammonium sulfate or the like, a desalting method, a precipitation method using an organic solvent, an anion exchange chromatography method using a resin such as diethylaminoethyl (DEAE)-sepharose or DIAION HPA-75 (manufactured by Mitsubishi Kasei Kogyo Kabushiki Kaisha), a cation exchange chromatography method using a resin such as S-sepharose FF (manufactured by Pharmacia Corporation), a hydrophobic chromatography method using a resin such as butyl sepharose and phenyl sepharose, a gel filtration method using a molecular sieve, an affinity chromatography method, a chromatofocusing method, an electrophoresis method such as isoelectric focusing phoresis and the like, alone or in combination thereof.

As the chromatography, column chromatography using phenyl-TOYOPEARL (Tosoh Corporation), DEAE-TOYOPEARL (Tosoh Corporation), and Sephadex G-150 (Pharmacia Biotech Inc.) is preferably used.

In a case where the spider silk protein is expressed with formation of an insoluble matter in cells, similarly, host cells are recovered, disrupted, and centrifuged to recover the insoluble matter of the spider silk protein as a precipitated fraction. The recovered insoluble matter of the spider silk protein can be solubilized with a protein denaturing agent. After this operation, a purified preparation of the spider silk protein can be obtained by the same isolation and purification method as described above.

In a case where the spider silk protein is secreted extracellularly, the spider silk protein can be recovered from a culture supernatant. That is, the culture supernatant is obtained by treating a culture by a technique such as centrifugation, and a purified preparation can be obtained from the culture supernatant by using the same isolation and purification method as described above.

A structural protein derived from collagen (collagen protein) includes a structural protein including a domain sequence represented by Formula 3: $[REP3]_p$ (in Formula 3, p represents an integer of 5 to 300, REP3 represents an amino acid sequence consisting of Gly-X-Y, and X and Y represent an optional amino acid residues other than Gly, and a plurality of REP3s may be the same or different amino acid sequences). Specifically, a structural protein including the amino acid sequence set forth in SEQ ID NO: 45 can be exemplified. The amino acid sequence set forth in SEQ ID NO: 45 is an amino acid sequence obtained by adding the amino acid sequence set forth in SEQ ID NO: 46 (tag sequence and hinge sequence) to the N-terminal of an amino acid sequence from the 301th residue to the 540th residue corresponding to repeated portions and motifs of a partial sequence of human collagen type 4 (NCBI Genbank Accession No.: CAA56335.1, GI: 3702452) obtained from the NCBI data base.

A structural protein derived from resilin (resilin protein) includes a structural protein including a domain sequence represented by Formula 4: $[REP4]_q$ (in Formula 4, q represents an integer of 4 to 300, REP4 represents an amino acid sequence consisting of Ser-J-J-Tyr-Gly-U-Pro, J represents an optional amino acid residue and is particularly preferably an amino acid residue selected from the group consisting of Asp, Ser, and Thr, U represents an optional amino acid residue and is particularly preferably an amino acid residue selected from the group consisting of Pro, Ala, Thr, and Ser, and a plurality of REP4s may be the same or different amino acid sequences). Specifically, a structural protein including the amino acid sequence set forth in SEQ ID NO: 47 can be exemplified. The amino acid sequence set forth in SEQ ID NO: 47 is an amino acid sequence obtained by adding the amino acid sequence set forth in SEQ ID NO: 46 (tag sequence and hinge sequence) to the N-terminal of an amino acid sequence from the 19th residue to the 321th residue, obtained by substituting Thr of the 87th residue with Ser and substituting Asn of the 95th residue with Asp in the amino acid sequence of resilin (NCBI Genbank Accession No.: NP 611157, Gl: 24654243).

Examples of a structural protein derived from elastin protein (elastin protein) include structural proteins having amino acid sequences such as NCBI Genbank Accession No. AAC98395 (human), I47076 (sheep), and NP786966 (cow). Specifically, a structural protein including the amino acid sequence set forth in SEQ ID NO: 48 can be exemplified. The amino acid sequence set forth in SEQ ID NO: 48 is an amino acid sequence obtained by adding the amino acid sequence set forth in SEQ ID NO: 46 (tag sequence and hinge sequence) to the N-terminal of an amino acid sequence from the 121th residue to the 390th residue of the amino acid sequence of NCBI Genbank Accession No. AAC98395.

As a structural protein derived from keratin (keratin protein), a type I keratin and the like of *Capra hircus* can be

exemplified. Specifically, a structural protein including the amino acid sequence set forth in SEQ ID NO: 49 (amino acid sequence of NCBI Genbank Accession No. ACY30466) can be exemplified. The amino acid sequence set forth in SEQ ID NO: 49 is an amino acid sequence obtained by adding the amino acid sequence set forth in SEQ ID NO: 46 (tag sequence and hinge sequence) to the N-terminal of the amino acid sequence of NCBI Genbank Accession No. ACY30466.

Examples of the silk fibroin include a structural protein including a domain sequence represented by the above Formula 1.

The collagen protein, resilin protein, elastin protein, keratin protein, and silk fibroin may be a hydrophilic protein or may be a hydrophobic protein. The hydrophobic protein is a protein in which a value (average HI) obtained by determining the total sum of hydropathy indices (HI) of all amino acid residues constituting the protein and then dividing the sum by the total number of amino acid residues is more than -0.8. The hydrophobic protein has preferably an average HI of -0.6 or more, more preferably -0.4 or more, still more preferably -0.2 or more, and particularly preferably 0 or more. The hydropathy index is as shown in Table 2. The hydrophilic protein is a protein having the above average HI of -0.8 or less.

The hydrophobic collagen protein, hydrophobic resilin protein, hydrophobic elastin protein, and hydrophobic keratin protein include a protein including the amino acid sequence set forth in SEQ ID NO: 45, SEQ ID NO: 48, or SEQ ID NO: 49 described above.

The hydrophilic collagen protein, hydrophilic resilin protein, hydrophilic elastin protein, and hydrophilic keratin protein include a protein including the amino acid sequence set forth in SEQ ID NO: 47.

The HI of each of the amino acid sequences set forth in SEQ ID NO: 45, SEQ ID NO: 47, SEQ ID NO: 48, and SEQ ID NO: 49 is as shown in Table 5. In the calculation of the HI of each amino acid sequence, calculation was performed excluding a sequence having no relationship with collagen protein, resilin protein, elastin protein, and keratin protein (that is, a sequence corresponding to the amino acid sequence set forth in SEQ ID NO: 12).

Also, the structural protein contains a hydrophobic protein and a polypeptide that tends to cause self-aggregation in a polar solvent, and is preferably a hydrophobic protein. One type of structural protein or structural protein derived therefrom can be used alone, or two or more types thereof can be used in combination. By combining two or more types of structural proteins, the entire hydrophobicity may be adjusted to a desired value. The hydrophobicity can be calculated by the method described above.

(Organic solvent)

As the organic solvent of the spinning dope according to the present embodiment, any organic solvent that can dissolve an artificial structural protein can be used. Examples of the organic solvent include hexafluoroisopropanol (HFIP), hexafluoroacetone (HFA), N,N-dimethylformamide (DMF), N,N-dimethylacetamide (DMA), 1,3-dimethyl-2-imidazolidone (DMI), N-methyl-2-pyrolidone (NMP), acetonitrile, N-methylmorpholine N-oxide (NMO), and formic acid. One type of these solvent may be used alone, or two or more types thereof can be mixed and used. For example, the organic solvent may contain at least one type selected from the group consisting of formic acid and HFIP, or may be formic acid. These organic solvents may contain water.

The concentration of the artificial structural protein in the spinning dope according to the present embodiment is preferably more than 10 to 50 by mass, more preferably 15 to 45% by mass, more preferably 20 to 45% by mass, more preferably 15 to 35% by mass, more preferably 20 to 40% by mass, still more preferably 20 to 35% by mass, still more preferably 25 to 35% by mass, and particularly preferably 28 to 34% by mass, based on 100% by mass of the total amount of the spinning dope. When the concentration of the artificial structural protein is more than 10% by mass, the spinnability of the spinning dope is improved, and the spinning dope can be more stably discharged from the spinneret. When the concentration of the artificial structural protein is 50% by mass or less, it is possible to avoid blocking of the hole of the spinneret when the spinning dope is discharged from the spinneret, thus further improving the productivity.

(Dissolution promoter)

The spinning dope according to the present embodiment may further contain a dissolution promoter. Inclusion of the dissolution promoter facilitates preparation of the spinning dope.

The dissolution promoter may be an inorganic salt composed of the following Lewis acid and Lewis base. Examples of the Lewis base include halide ions. Examples of the Lewis acid include metal ions such as alkaline metal ions, and halide alkaline earth metal ions. Examples of the inorganic salt include alkaline metal halides, and alkaline earth metal halides. Specific examples of the alkaline metal halide include lithium chloride and lithium bromide. Specific examples of the alkaline earth halide include magnesium chloride and calcium chloride. One type of dissolution promoter can be used alone, or two or more types thereof can be used in combination.

These inorganic salts can be used as a dissolution promoter for structural protein against formic acid or DMSO, and lithium chloride and calcium chloride are particularly preferable. Inclusion of the dissolution promoter (the above inorganic salts) in the spinning dope allows the structural protein to be dissolved at a high concentration in the spinning

dope. With this, the production efficiency of the protein fiber is further improved, and improvement in quality of the protein fiber, improvement of physical properties such as stress, and the like can be expected.

The content of the dissolution promoter may be 0.1% by mass or more, 1% by mass or more, 2% by mass or more, 3% by mass or more, 4% by mass or more, 7% by mass or more, 10% by mass or more, or 15% by mass or more; or may be 20% by mass or less, 16% by mass or less, 12% by mass or less, or 9% by mass or less, based on 100 % by mass of the total amount of the spinning dope.

(Various additives)

The spinning dope may further contain various types of additives as necessary. Examples of the additive include a plasticizer, a leveling agent, a crosslinking agent, a nucleating agent, an antioxidant, an ultraviolet absorber, a coloring agent, a filler, and a synthetic resin. The content of the additive may be 50 parts by mass or less based on 100 parts by mass of the total amount of protein in the spinning dope.

The viscosity of the spinning dope according to the present embodiment may be appropriately set depending on the application of the fiber, the spinning method, and the like. The viscosity at 20°C may be 60,000 to 130,000 mPa·sec, or may be 65,000 to 125,000 mPa·sec, for example. The viscosity at 35°C may be 500 to 35,000 mPa·sec, 1,000 to 35,000 mPa·sec, 3,000 to 30,000 mPa·sec, 500 to 20,000 mPa·sec, or 500 to 15,000 mPa·sec, or may be 1,000 to 15,000 mPa·sec, 1,000 to 12,000 mPa·sec, 1,500 to 12,000 mPa·sec, 1,500 to 10,000 mPa·sec, or 1,500 to 8,000 mPa·sec, for example. The viscosity at 40°C may be 500 to 35,000 mPa·sec, 1,000 to 35,000 mPa·sec, 5,000 to 35,000 mPa·sec, 10,000 to 30,000 mPa·sec, or 5,000 to 20,000 mPa·sec, or may be 8,000 to 20,000 mPa·sec, 9,000 to 18,000 mPa·sec, 9,000 to 16,000 mPa·sec, 10,000 to 15,000 mPa·sec, 12,000 to 30,000 mPa·sec, 12,000 to 28,000 mPa·sec, 12,000 to 18,000 mPa·sec, or 12,000 to 16,000 mPa·sec, for example. The viscosity at 70°C may be 1,000 to 6,000 mPa·sec, or 1,500 to 5,000 mPa·sec, for example. The viscosity of the spinning dope can be measured by using, for example, an "EMS viscometer" (trade name) manufactured by Kyoto Electronics Manufacturing Co., Ltd.

The spinning dope may be stirred or shaken for a certain period of time in order to promote dissolution. At this time, the spinning dope may be heated, as necessary, to a temperature at which the spinning dope can be dissolved, depending on a structural protein and solvent to be used. The spinning dope may be heated to 30°C or more, 40°C or more, 50°C or more, 60°C or more, 70°C or more, 80°C or more, or 90°C or more, for example. From the viewpoint of preventing decomposition of the modified fibroin, the heating temperature is preferably 40°C. The upper limit of the heating temperature is, for example, a temperature equal to or less than the boiling point of the solvent.

<Coagulation liquid>

The coagulation liquid according to the present embodiment is not particularly limited, but preferably contains water or an aqueous solution having a PH of 0.25 or more and 10.00 or less. Inclusion of water or an aqueous solution having a PH of 0.25 or more and 10.00 or less makes it possible to provide a method for producing a protein fiber in which the risk of explosion, fire, or the like, production cost, and environmental load are reduced. The aqueous solution may be a salt aqueous solution, an acid aqueous solution, or a mixed solution of a salt aqueous solution and an acid aqueous solution, may be a salt aqueous solution, or a mixed solution of a salt aqueous solution and an acid aqueous solution, or may be a salt aqueous solution. Here, the mixed solution of a salt aqueous solution and an acid aqueous solution is not limited to a solution in which a salt aqueous solution and an acid aqueous solution are mixed. The mixed solution includes a solution in which an acid is added to a salt aqueous solution, a solution in which a salt is added to an acid aqueous solution, and a solution in which a salt and an acid are dissolved in water.

(Acid aqueous solution)

Examples of the acid aqueous solution include aqueous solutions of carboxylic acid, and the like. Specific examples of the carboxylic acid include formic acid, acetic acid, propionic acid, citric acid, and oxalic acid. One type of these solvent may be used alone, or two or more types thereof may be mixed and used as an aqueous solution. The acid aqueous solution may be, for example, a citric acid aqueous solution or a formic acid aqueous solution.

(Salt aqueous solution)

Examples of the salt aqueous solution include a salt aqueous solution of an organic salt or an inorganic salt, and a mixed aqueous solution of an organic salt and an inorganic salt.

Examples of the organic salt include carboxylate and the like. Specific examples of the carboxylate include a formate, an acetate, a propionate, a citrate, and an oxalate. The organic salt may be, for example, a formate, an acetate, and a citrate.

Specific examples of the formate include ammonium formate, potassium formate, sodium formate, lithium formate, magnesium formate, and calcium formate.

Specific examples of the acetate include ammonium acetate, potassium acetate, sodium acetate, lithium acetate, magnesium acetate, and calcium acetate.

Specific examples of the propionate include ammonium propionate, potassium propionate, sodium propionate, lithium propionate, magnesium propionate, and calcium propionate.

Specific examples of the citrate include ammonium citrate, potassium citrate, sodium citrate, lithium citrate, magnesium citrate, and calcium citrate. For example, the citrate may include at least one type selected from the group consisting of ammonium citrate, potassium citrate, sodium citrate, magnesium citrate, and calcium citrate. The citrate may include at least one type selected from the group consisting of ammonium citrate, potassium citrate, and sodium citrate. The citrate may include at least one type selected from the group consisting of potassium citrate and sodium citrate. The citrate may be sodium citrate.

Specific examples of the oxalate include ammonium oxalate, potassium oxalate, sodium oxalate, lithium oxalate, magnesium oxalate, and calcium oxalate. The carboxylate is more preferably a sodium carboxylate, and specific examples of the sodium carboxylate include sodium formate, sodium acetate, sodium propionate, and sodium oxalate.

Specific examples of the inorganic salt include a normal salt, an acid salt, and a basic salt.

Specific examples of the normal salt include a sulfate, a chloride, a nitrate, an iodide salt, a thiocyanate, and a carbonate.

Specific examples of the sulfate include ammonium sulfate, potassium sulfate, sodium sulfate, lithium sulfate, magnesium sulfate, and calcium sulfate. For example, the sulfate may include at least one type selected from the group consisting of ammonium sulfate, sodium sulfate, magnesium sulfate, and calcium sulfate. The sulfate may include at least one type selected from the group consisting of ammonium sulfate and sodium sulfate. The sulfate may be sodium sulfate.

Specific examples of the chloride include ammonium chloride, potassium chloride, sodium chloride, lithium chloride, magnesium chloride, and calcium chloride. For example, the chloride may include at least one type selected from the group consisting of ammonium chloride, potassium chloride, sodium chloride, lithium chloride, calcium chloride, and magnesium chloride. The chloride may include at least one type selected from the group consisting of potassium chloride, sodium chloride, and calcium chloride. The chloride may include at least one type selected from the group consisting of sodium chloride and calcium chloride. The chloride may be sodium chloride.

Specific examples of the nitrate include ammonium nitrate, potassium nitrate, sodium nitrate, lithium nitrate, magnesium nitrate, and calcium nitrate.

Specific examples of the iodide salt include ammonium iodide, potassium iodide, sodium iodide, lithium iodide, magnesium iodide, and calcium iodide.

Specific examples of the thiocyanate include ammonium thiocyanate, potassium thiocyanate, sodium thiocyanate, lithium thiocyanate, magnesium thiocyanate, calcium thiocyanate, and guanidine thiocyanate.

Specific examples of the carbonate include ammonium carbonate, potassium carbonate, sodium carbonate, lithium carbonate, magnesium carbonate, and calcium carbonate.

Specific examples of the acid salt include a hydrogen sulfate, a hydrogen phosphate, and a bicarbonate.

Specific examples of the hydrogen sulfate include ammonium hydrogen sulfate, potassium hydrogen sulfate, sodium hydrogen sulfate, lithium hydrogen sulfate, magnesium hydrogen sulfate, and calcium hydrogen sulfate.

Specific examples of the hydrogen phosphate include sodium dihydrogen phosphate, disodium hydrogen phosphate, potassium dihydrogen phosphate, dipotassium hydrogen phosphate, ammonium dihydrogen phosphate, ammonium dihydrogen phosphate, diammonium hydrogen phosphate, magnesium dihydrogen phosphate, dimagnesium hydrogen phosphate, calcium dihydrogenphosphate, and dicalcium hydrogen phosphate.

Specific examples of the bicarbonate include ammonium bicarbonate, potassium bicarbonate, sodium bicarbonate, lithium bicarbonate, lithium bicarbonate, magnesium bicarbonate, and calcium bicarbonate.

Specific examples of the basic salt include calcium hydroxide chloride, and magnesium hydroxide chloride.

One type of the above acid, acid aqueous solution, salt, and salt aqueous solution may be used alone, or two or more types thereof can be mixed and used.

Examples of the salt mixed aqueous solution in which two or more types of salts or salt aqueous solutions are mixed include a mixed aqueous solution of the organic salts, a mixed aqueous solution of the inorganic salts, and a mixed aqueous solution of the organic salt and the inorganic salt. Brackish water and sea water are particularly preferable from the viewpoint of reducing production cost. The brackish water and sea water are known to primarily contain potassium chloride, sodium chloride, magnesium chloride, magnesium sulfate, and calcium sulfate.

Preferably, the coagulation liquid preferably contains a salt aqueous solution, and more preferably, the coagulation liquid is a salt aqueous solution. Inclusion of salt can further improve solvent removal rate. The salt more preferably includes at least one type selected from the group consisting of a carboxylate, a sulfate, a chloride, a hydrogen phosphate, and a bicarbonate. The salt still more preferably includes at least one type selected from the group consisting of a carboxylate, a sulfate, and a chloride, and still more preferably includes at least one type selected from the group

consisting of a sulfate and a chloride. The salt particularly preferably includes a sulfate. Inclusion of these salts can further improve the fiber-forming property, and thus can further improve elongation of the fiber to be obtained.

The carboxylate is more preferably sodium carboxylate. The sulfate is more preferably ammonium sulfate, sodium sulfate, magnesium sulfate, and calcium sulfate. The chloride is more preferably potassium chloride, sodium chloride, magnesium chloride, and calcium chloride. The bicarbonate is more preferably sodium bicarbonate. The mixed aqueous solution is particularly preferably brackish water and sea water. Use of these salts and mixed aqueous solutions can further reduce production cost, in addition to the effect of improving the fiber-forming property.

The content of the salt may be 0.1% by mass or more, 0.3% by mass or more, 0.5% by mass or more, 0.7% by mass or more, 1% by mass or more, 1.3% by mass or more, 1.5% by mass or more, 1.7% by mass or more, 2% by mass or more, 2.3% by mass or more, 2.5% by mass or more, 2.7% by mass or more, 3% by mass or more, 4% by mass or more, 5% by mass or more, 7% by mass or more, 10% by mass or more, 15% by mass or more, or 20% by mass or more, based on the total amount of the coagulation liquid. The upper limit thereof may be 30% by mass or less, 25% by mass or less, or less than or equal to the solubility. The content of the salt may be, for example, 0.1% by mass or more and 30% by mass or less, 0.3% by mass or more and 25% by mass or less, 1% by mass or more and 25% by mass or less, 3% by mass or more and 25% by mass or less, 5% by mass or more and 25% by mass or less, 8% by mass or more and 25% by mass or less, 10% by mass or more and 25% by mass or less, 12% by mass or more and 25% by mass or less, 1% by mass or more and 20% by mass or less, 3% by mass or more and 20% by mass or less, 5% by mass or more and 20% by mass or less, 8% by mass or more and 20% by mass or less, 10% by mass or more and 20% by mass or less, 10% by mass or more and 15% by mass or less, 12% by mass or more and 20% by mass or less, 12% by mass or more and 18% by mass or less, 12% by mass or more and 17% by mass or less, 12% by mass or more and 16% by mass or less, 15% by mass or more and 20% by mass or less, or 16% by mass or more and 20% by mass or less, based on the total amount of the coagulation liquid. The content of the salt is, for example, preferably 0.05 mol/L or more, and may be 0.05 mol/L or more and 5.5 mol/L or less, 0.1 mol/L or more and 5.0 mol/L or less, 0.1 mol/L or more and 4.5 mol/L or less, or 0.1 mol/L or more and 4.0 mol/L or less, based on the total amount of the coagulation liquid.

The content of the salt in a case of using sodium chloride may be, for example, 0.1 mol/L or more and 5.0 mol/L or less, 0.1 mol/L or more and 4.5 mol/L or less, or 0.1 mol/L or more and 4.0mol/L or less, based on the total amount of the coagulation liquid.

The content of the salt in a case of using potassium chloride may be, for example, 0.1 mol/L or more and 3.9 mol/L or less, based on the total amount of the coagulation liquid.

The content of the salt in a case of using calcium chloride may be, for example, 0.1 mol/L or more and 14.3 mol/L or less, 0.1 mol/L or more and 13.0 mol/L or less, 0.1 mol/L or more and 12.0 mol/L or less, 0.1 mol/L or more and 11.0 mol/L or less, 0.1 mol/L or more and 10.0 mol/L or less, 0.1 mol/L or more and 9.0 mol/L or less, 0.1 mol/L or more and 8.0 mol/L or less, 0.1 mol/L or more and 7.0 mol/L or less, 0.1 mol/L or more and 6.0 mol/L or less, 0.1 mol/L or more and 5.0 mol/L or less, 0.1 mol/L or more and 4.0 mol/L or less, 0.1 mol/L or more and 3.0 mol/L or less, or 0.1 mol/L or more and 2.0 mol/L or less, based on the total amount of the coagulation liquid.

The content of the salt in a case of using a case of using sodium sulfate may be, for example, 0.1 mol/L or more and 3.4 mol/L or less, 0.1 mol/L or more and 3.0 mol/L or less, 0.1 mol/L or more and 2.5 mol/L or less, or 0.1 mol/L or more and 2.0 mol/L or less, based on the total amount of the coagulation liquid. The content may be, for example, 3% by mass or more and 28% by mass or less, 3% by mass or more and 25% by mass or less, 3% by mass or more and 20% by mass or less, 5% by mass or more and 20% by mass or less, 8% by mass or more and 20% by mass or less, 10% by mass or more and 20% by mass or less, 10% by mass or more and 18% by mass or less, 12% by mass or more and 20% by mass or less, 12% by mass or more and 18% by mass or less, 12% by mass or more and 16% by mass or less, or 13% by mass or more and 16% by mass or less, based on the total amount of the coagulation liquid.

The content of the sodium sulfate relative to the total amount of the coagulation liquid is preferably 10% by mass or more and 20% by mass or less, preferably 11% by mass or more and 19% by mass or less, more preferably 11% by mass or more and 18% by mass or less, still more preferably 12% by mass or more and 18% by mass or less, still more preferably 12% by mass or more and 17% by mass or less, still more preferably 13% by mass or more and 17% by mass or less, and particularly preferably 13% by mass or more and 16% by mass or less. When the content of the sodium sulfate relative to the total amount of the coagulation liquid is 10% by mass or more, coagulation rate is further increased, thus enabling further reduction in cost increase due to facility investment. When the content of the sodium sulfate relative to the total amount of the coagulation liquid is 20% by mass or less, breakage of the yarn can be further reduced that occurs at the interface between the dope solution and the coagulated yarn (thread) caused by rapid coagulation of the dope solution.

The content of water relative to the total amount of the coagulation liquid in the above case is preferably 50% by mass or more and 80% by mass or less, more preferably 60% by mass or more and 80% by mass or less, and still more preferably 60% by mass or more and 70% by mass or less from the viewpoint of improving the recovery efficiency of the dope solvent.

The concentration of the sodium sulfate aqueous solution in a case of using sodium sulfate is preferably 10% by mass or more and 22% by mass or less, preferably 10% by mass or more and 20% by mass or less, more preferably 12% by

mass or more and 20% by mass or less, still more preferably 14% by mass or more and 20% by mass or less, and particularly preferably 16% by mass or more and 20% by mass or less. When the concentration of the sodium sulfate aqueous solution is 10% by mass or more, sufficient coagulation rate can be obtained, thus enabling further reduction in cost increase due to facility investment. When the concentration of the sodium sulfate aqueous solution is 22% by mass or less, breakage of the yarn can be further reduced that occurs at the interface between the dope solution and the coagulated yarn (thread) caused by rapid coagulation of the dope solution.

The content of the salt in a case of using sodium citrate may be, for example, 0.1 mol/L or more and 1.6 mol/L or less, or 0.1 mol/L or more and 1.3 mol/L or less, based on the total amount of the coagulation liquid.

The aqueous solution contained in the coagulation liquid of the present embodiment may be selected from the group consisting of, for example, a carboxylic acid aqueous solution, a bicarbonate aqueous solution, a formate aqueous solution, an acetate aqueous solution, a chloride aqueous solution, a sulfate aqueous solution, a hydrogen phosphate aqueous solution, a citrate aqueous solution, brackish water, sea water, and a mixed solution thereof. The aqueous solution contained in the coagulation liquid of the present embodiment may be, for example, selected from the group consisting of a citric acid aqueous solution, a formic acid aqueous solution, a sodium bicarbonate aqueous solution, a sodium formate aqueous solution, a sodium acetate aqueous solution, a sodium chloride aqueous solution, a sodium sulfate aqueous solution, an ammonium sulfate aqueous solution, a potassium hydrogen phosphate aqueous solution, a calcium chloride aqueous solution, a sodium citrate aqueous solution, brackish water, sea water, and a mixed solution thereof. The aqueous solution may be at least one type selected from the group consisting of a sodium chloride aqueous solution, a sodium citrate aqueous solution, a sodium sulfate aqueous solution, brackish water, sea water, and a mixed solution thereof. The aqueous solution may be at least one type selected from the group consisting of a sodium chloride aqueous solution, a sodium citrate aqueous solution, a sodium sulfate aqueous solution, and a mixed solution thereof. The aqueous solution may be at least one type selected from the group consisting of a sodium chloride aqueous solution, a sodium citrate aqueous solution, and a sodium sulfate aqueous solution.

Further, from the viewpoint of the fiber-forming property, the aqueous solution contained in the coagulation liquid of the present embodiment is preferably a salt aqueous solution (Table 8). The salt aqueous solution is preferably at least one type selected from the group consisting of a sulfate aqueous solution, a chloride aqueous solution, a carboxylate aqueous solution, a hydrogen phosphate aqueous solution, a bicarbonate aqueous solution, brackish water, and sea water. The sulfate is preferably at least one type selected from the group consisting of ammonium sulfate, potassium sulfate, sodium sulfate, lithium sulfate, magnesium sulfate, and calcium sulfate. The chloride is preferably at least one type selected from the group consisting of sodium chloride, calcium chloride, ammonium chloride, potassium chloride, lithium chloride, and magnesium chloride. Further, the salt aqueous solution is more preferably at least one type selected from the group consisting of a sodium chloride aqueous solution, a sodium sulfate aqueous solution, and a sodium citrate aqueous solution.

The coagulation liquid before contact with the spinning dope may or may not contain an organic solvent. When the coagulation liquid contains an organic solvent, the organic solvent may be the same as or different from the organic solvent in the spinning dope, but is preferably the same. Even in a case where the coagulation liquid before contact with the spinning dope contains no organic solvent, there may be a case where the organic solvent is dissolved from the spinning dope in contact with the coagulation liquid in the coagulation liquid in a process of bringing the spinning dope into contact with the coagulation liquid. The content of the organic solvent contained in the coagulation liquid (including a case where the organic solvent is dissolved from the spinning dope in contact with the coagulation liquid to the coagulation liquid) may be 0% by mass or more and 30% by mass or less, 5% by mass or more and 30% by mass or less, 5% by mass or more and 25% by mass or less, 0% by mass or more and 20% by mass or less, 5% by mass or more and 20% by mass or less, 5% by mass or more and 15% by mass or less, 10% by mass or more and 30% by mass or less, 10% by mass or more and 20% by mass or less, 0% by mass or more and 10% by mass or less, 0% by mass or more and 5% by mass or less, or 0% by mass or more and 2% by mass or less, preferably 10% by mass or more and 30% by mass or less, more preferably 12% by mass or more and 28% by mass or less, more preferably 14% by mass or more and 26% by mass or less, more preferably 15% by mass or more and 25% by mass or less, still more preferably 18% by mass or more and 24% by mass or less, and particularly preferably 18% by mass or more and 22% by mass or less, based on 100% by mass of the total amount of the coagulation liquid (in a case where the organic solvent is dissolved from the spinning dope to the coagulation liquid, the total content of the coagulation liquid before contact with the spinning dope and the organic solvent dissolved from the spinning dope to the coagulation liquid). When the content of the organic solvent is within the above-described range, the fiber-forming property of the structural protein is further improved. As the organic solvent, formic acid, DMSO, or HFIP is preferable, formic acid or HFIP is more preferable, and formic acid is still more preferable.

The pH of the aqueous solution contained in the coagulation liquid may be 0.25 to 10.00, or 0.25 to 9.50.

The pH of the acid aqueous solution in the coagulation liquid may be, for example, less than 0.25 to 7.00, less than 0.50 to 7.00, less than 1.00 to 7.00, less than 1.50 to 7.00, less than 2.00 to 7.00, or less than 3.00 to 7.00.

The pH of the salt aqueous solution in the coagulation liquid may be, for example, 0.50 to 10.00, 1.00 to 10.00, 2.00 to 10.00, 3.00 to 10.00, 3.50 to 10.00, 4.00 to 10.00, 4.50 to 10.00, 5.00 to 10.00, 5.50 to 10.00, 6.00 to 10.00, 6.50 to

10.00, or 6.50 to 9.50.

The content of the water or aqueous solution in the coagulation liquid is preferably 60% by mass or more, more preferably 65% by mass or more, more preferably 68% by mass or more, more preferably 70% by mass or more, more preferably 71% by mass or more, more preferably 72% by mass or more, more preferably 73% by mass or more, more preferably 74% by mass or more, more preferably 75% by mass or more, more preferably 76% by mass or more, more preferably 77% by mass or more, more preferably 78% by mass or more, still more preferably 79% by mass or more, particularly preferably 80% by mass or more, and may be 85% by mass or more, 90% by mass or more, or 95% by mass, based on 100% by mass of the total amount of the coagulation liquid. When the content of the water or the aqueous solution is within the above-described range, the fiber-forming property of the structural protein is further improved. The content of the water or aqueous solution in the coagulation liquid may be, for example, 60% by mass or more and 100% by mass or less, 70% by mass or more and 100% by mass or less, 75% by mass or more and 100% by mass or less, 80% by mass or more and 100% by mass or less, 85% by mass or more and 100% by mass or less, 90% by mass or more and 100% by mass or less, 95% by mass or more and 100% by mass or less, 70% by mass or more and 90% by mass or less, 75% by mass or more and 85% by mass or less, or 78% by mass or more and 82% by mass or less, based on the total amount of the coagulation liquid.

The temperature of the coagulation liquid may be room temperature, 0°C to 90°C, 0°C to 80°C, 5°C to 80°C, 10°C to 80°C, 15°C to 80°C, 20°C to 80°C, 25°C to 80°C, 30°C to 80°C, 40°C to 80°C, 50°C to 80°C, 60°C to 80°C, 70°C to 80°C, 20°C to 70°C, 30°C to 70°C, 40°C to 70°C, 50°C to 70°C, 20°C to 60°C, 30°C to 60°C, 40°C to 60°C, or 50°C to 60°C. The temperature of the coagulation liquid is preferably 30°C to 50°C, more preferably 32°C to 48°C, more preferably 33°C to 47°C, more preferably 34°C to 46°C, and still more preferably 35°C to 45°C from the viewpoint of better spinning stability. The lower limit of the temperature of the coagulation liquid may be equal to or more than the melting point of the organic solvent contained in the spinning dope. The upper limit of the temperature may be equal to or less than the boiling point of the organic solvent contained in the spinning dope. By setting the temperature of the coagulation liquid to a higher temperature, the solvent removal rate of the spinning dope can be increased.

The coagulation liquid may further contain a dope solvent (e.g., formic acid). The content of the dope solvent relative to the total amount of the coagulation liquid is preferably 15 to 25% by mass, more preferably 16 to 25% by mass, still more preferably 16 to 24% by mass, and particularly preferably 18 to 24% by mass from the viewpoint of improving the recovery efficiency of the solvent.

The coagulation liquid may further contain the above-described dissolution promoter that can be added to the spinning dope.

[Spinning process]

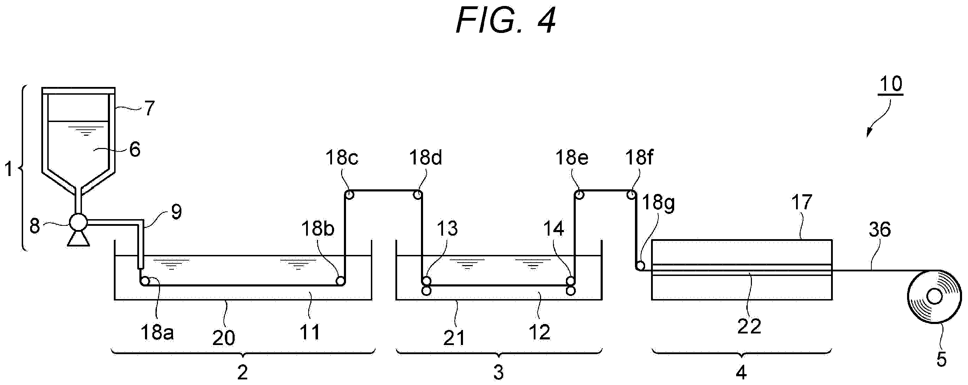

The artificial structural protein fiber according to the present embodiment can be produced by a publicly known wet spinning method. Examples thereof include a method including a process (coagulation process) of discharging a spinning dope containing an artificial structural protein and an organic solvent from a spinneret into a coagulation liquid to coagulate the artificial structural protein. Here, in the coagulation process, the spinning draft (bath draft) is more than 0.7 and 20 or less. When the spinning draft (bath draft) is more than 0.7, the diameter of the fiber can be further reduced. The method for producing a protein fiber of the present embodiment can be performed by, for example, using the spinning apparatus shown in Fig. 4.

Fig. 4 is an explanatory view illustrating an example of a spinning apparatus for producing a protein fiber. The spinning apparatus 10 shown in Fig. 4 is an example of the spinning apparatus for wet spinning, and has an extrusion apparatus 1, a coagulation bath 20, a washing bath (drawing bath) 21, and a drying apparatus 4 from the upstream side in this order.

The extrusion apparatus 1 has a storage tank 7 that stores a spinning dope (dope solution) 6. The coagulation bath 20 stores a coagulation liquid 11. The spinning dope 6 is extruded from a nozzle 9 provided in the coagulation liquid 11 by a gear pump 8 attached to the lower end portion of the storage tank 7. The extruded spinning dope 6 is supplied (introduced) to the coagulation liquid 11 in the coagulation bath 20. The solvent is removed from the spinning dope in the coagulation liquid 11, and the spider silk protein coagulates. The coagulated spider silk protein is introduced into a washing bath 21, washed with a washing solution 12 in the washing bath 21, then sent to the drying apparatus 4 by a first nip roller 13 and a second nip roller 14 provided in the washing bath 21. At this time, when the rotational speed of the second nip roller 14 is made faster than the rotational speed of the first nip roller 13, for example, a protein fiber 36 drawn at a ratio corresponding to the rotational speed ratio is obtained. The protein fiber drawn in the washing solution 12 is taken out from the washing bath 21, dried at the time of passing through inside of the drying apparatus 4, and then wound by a winder. In this way, in the spinning apparatus 10, the protein fiber is finally wound as a wound roll 5 by the winder. Incidentally, reference numerals 18a to 18g are yarn guides.

The temperature of the coagulation liquid 11 is not particularly limited, but may be 55°C or less, 50°C or less, 45°C or less, 40°C or less, 30°C or less, 25°C or less, 20°C or less, 10°C or less, or 5°C or less. The temperature is preferably 0°C

or more from the viewpoint of workability, cooling cost, and the like. Additionally, the temperature of the coagulation liquid 11 can be adjusted by, for example, using the spinning apparatus 10 having the coagulation bath 20 including a heat exchanger inside thereof and a coolant circulation device. For example, a cooled medium, which has been cooled to a predetermined temperature by the coolant circulation device, is allowed to flow through the heat exchanger provided in the coagulation bath. Whereby, the temperature can be adjusted to a temperature within the above range by heat exchange between the coagulation liquid 11 and the heat exchanger. In this case, more efficient cooling can be achieved by circulating, as a medium, a solvent used for the coagulation liquid 11.

A plurality of coagulation baths storing the coagulation liquid may be provided.

The coagulated artificial structural protein removed from the coagulation bath or the washing bath may be wound as is by the winder, or may be dried by being allowed to pass through the drying apparatus and then wound by the winder.

The distance for which the coagulated artificial structural protein passes through the coagulation liquid may be any distance as long as the solvent can be efficiently removed. The distance may be determined depending on, for example, the extrusion speed (discharge speed) of the spinning dope from the nozzle. The residence time of the coagulated artificial structural protein (or spinning dope) in the coagulation liquid may be determined depending on the distance for which the coagulated artificial structural protein passes through the coagulation liquid, the extrusion speed of the spinning dope from the nozzle, and the like.

The term "spinning draft (bath draft)" means a value obtained by dividing a speed (take-up speed) at which the coagulated yarn is taken up by a take-up roller (godet roller) 18b by a linear velocity (discharge linear velocity) at which the spinning dope is discharged from the spinneret. The discharge linear velocity and the take-up speed may be appropriately adjusted according to physical properties such as a desired fiber diameter, the production amount, and the like.

The value of the spinning draft (bath draft) can be appropriately adjusted according to the hole diameter of the spinneret to be used. For example, when a spinneret having a hole diameter of 0.04 mm to 0.1 mm is used, the value is more than 0.7 to 20 times, more preferably more than 0.8 to 20 times, more preferably 0.8 to 15 times, more preferably 0.8 to 10 times, more preferably 1 to 7 times, more preferably 2 to 7 times, more preferably 2 to 6.5 times, still more preferably 3 to 6.5 times, and particularly preferably 3 to 6 times. The value may be more than 0.7 to 12 times, 0.7 to 10 times, may be 0.7 to 5 times, 0.7 to 4.5 times, 0.8 to 10 times, more than 0.8 to 10 times, 0.8 to 9 times, more than 0.8 to 9 times, 0.8 to 8 times, more than 0.8 to 8 times, 0.8 to 7 times, more than 0.8 to 7 times, 0.8 to 6.5 times, more than 0.8 to 6.5 times, or more than 0.8 to 6 times, may be 1 to 10 times, 1 to 9 times, 1 to 8 times, 1 to 7 times, 1 to 6.5 times, 1 to 6 times, or 1 to 5 times, may be 1.2 to 10 times, 1.2 to 9 times, 1.2 to 8 times, 1.2 to 7 times, 1.2 to 6.5 times, 1.2 to 6 times, or 1.2 to 5 times, may be 1.5 to 10 times, 1.5 to 9 times, 1.5 to 8 times,1.5 to 7 times,1.5 to 6.5 times,1.5 to 6 times, 1.5 to 5.5 times, or 1.5 to 5 times, may be 1.8 to 10 times,1.8 to 9 times, 1.8 to 8 times, 1.8 to 7 times,1.8 to 6.5 times, 1.8 to 6 times, 1.8 to 5.5 times, or 1.8 to 5 times, may be 2 to10 times, 2 to 9 times, 2 to 8 times, 2 to 6 times, 2 to 5.5 times, or 2 to 5 times, may be 2.5 to 10 times, 2.5 to 9 times, 2.5 to 8 times, 2.5 to 7 times, 2.5 to 6.5 times, 2.5 to 6 times, 2.5 to 5.5 times, or 2.5 to 5 times, may be 3 to10 times, 3 to 9 times, 3 to 8 times, 3 to 7 times, 3 to 5.5 times, or 3 to 5 times, or may be 3.5 to10 times, 3.5 to 9 times, 3.5 to 8 times, 3.5 to 7 times, 3.5 to 6 times, 3.5 to 5.5 times, or 3.5 to 5 times. When the value of the spinning draft (bath draft) is more than 0.7, the spinning stability is further improved, and the productivity can be improved. When the value of the spinning draft (bath draft) is 20 times or less, the equipment cost can be further reduced, and the fiber diameter reduction effect and the stress improvement effect can be sufficiently obtained.

[Drawing process]

The method for producing an artificial structural protein of the present embodiment may further include a process of drawing the coagulated artificial structural protein fiber (drawing process). Examples of the drawing method include wet heat drawing, dry heat drawing and the like. The drawing process may be performed by, for example, in the coagulation bath 20, or in the washing bath 21. The drawing process can also be performed in the air.

The drawing in the washing bath 21 may be drawing in hot water, in a solution in which an organic solvent is added to hot water, or the like, that is, wet heat drawing. The temperature for wet heat drawing is preferably 50 to 90°C. When this temperature is 50°C or more, the pore diameter of the yarn can be stably made small. Also, when the temperature is 90°C or less, temperature setting is easy, and thus spinning stability is improved. The temperature is more preferably 75 to 85°C.

The wet heat drawing can be performed in hot water, in a solution in which an organic solvent or the like is added to hot water, or in a heated steam. The temperature may be, for example, 40 to 200°C, 50 to 180°C, 50 to 150°C, or 75 to 90°C. The draw ratio in the wet heat drawing may be, for example, 1 to 30 times, 2 to 25 times, 2 to 20 times, 2 to 15 times, 2 to 10 times, 2 to 8 times, 2 to 6 times, or 2 to 4 times, with respect to the undrawn yarn (or pre-drawing yarn). However, the draw ratio is not limited as long as characteristics such as a desired fiber thickness and mechanical properties can be obtained.

The dry heat drawing can be performed by using an apparatus such as a contact-type hot plate and a noncontact type furnace, but is not particularly limited thereto. Any apparats can be used that increases the temperature of the fiber to a desired temperature and allows drawing at a predetermined draw ratio. The temperature for dry heat drawing may be, for example, 100°C to 270°C, 140°C to 230°C, 140°C to 200°C, 160°C to 200°C, or 160°C to 180°C.

The draw ratio in the dry heat drawing process may be, for example, 1 to 30 times, may be 2 to 30 times, may be 2 to 20 times, may be 3 to 15 times, preferably 3 to 10 times, more preferably 3 to 8 times, and still more preferably 4 to 8 times, with respect to the undrawn yarn (or pre-drawing yarn). However, the draw ratio is not limited as long as characteristics such as a desired fiber thickness and mechanical properties can be obtained.

The drawing process may be a process that performs each of wet heat drawing and dry heat drawing separately, or a process that performs these drawings in multiple stages or in combination. That is, as the drawing process, wet heat drawing and dry heat drawing can be appropriately combined and performed as follows: wet heat drawing is performed at a first drawing stage and then dry heat drawing is performed at a second drawing stage, or wet heat drawing is performed at a first drawing stage, then wet heat drawing is performed at a second drawing stage, and further dry heat drawing is performed at a third drawing stage, for example.

The lower limit of the final draw ratio of the artificial structural protein fiber subjected to the drawing process may be preferably any of 1 time, 2 times, 3 times, 4 times, 5 times, 6 times, 7 times, 8 times, and 9 times, with respect to the undrawn yarn (or pre-drawing yarn). The upper limit of the final draw ratio of the modified fibroin fiber subjected to the drawing process may be preferably any of 40 times, 30 times, 20 times, 15 times, 14 times, 13 times, 12 times, 11 times, and 10 times. The final draw ratio may be, for example, 3 to 40 times, 3 to 30 times, 5 to 30 times, 5 to 20 times, 5 to 15 times, or 5 to 13 times. However, the draw ratio is not limited as long as characteristics such as a desired fiber thickness and mechanical properties can be obtained.

In the spinning process, the shape of the spinneret, the shape of the hole, the number of holes is not particularly limited, and can be appropriately selected depending on a desired fiber diameter, the number of single yarns, and the like.

An oil agent may be applied to an undrawn yarn (or pre-drawing yarns) or drawn yarn, as necessary, for the purpose of imparting an antistatic property, convergence and lubricity, or the like before or after drying. The type of the oil agent applied and application amount thereof, and the like are not particularly limited, and can be appropriately adjusted in consideration of use application of the fiber, dealing of the fiber, and the like.

In a case where the hole shape of the spinneret is a circular shape, the hole diameter of the spinneret may be 0.01 mm or more and 0.6 mm or less, for example. When the hole diameter is 0.01 mm or more, the pressure loss can be reduced, and thus the facility cost can be suppressed. When the hole diameter is 0.6 mm or less, the necessity of the drawing operation for minimizing the fiber diameter can be reduced. Thus, a possibility of causing breakage on drawing during a period from discharge to winding can be reduced.

The temperature of the spinning dope when the spinning dope passes through the spinneret and the temperature of the spinneret are not particularly limited. The temperatures may be appropriately adjusted depending on the concentration and viscosity of the spinning dope to be used, the type of the organic solvent, and the like. The temperatures are preferably 30°C to 100°C from the viewpoint of preventing deterioration the structural protein, for example. Also, the upper limit of the temperature is preferably a temperature that does not reach the boiling point of a solvent to be used, from the viewpoint of reducing possibilities of pressure increase due to volatilization of the solvent and clogging in the conduit due to solidification of the spinning dope. This improves process stability.

The production method according to the present embodiment may further include a process of filtrating the spinning dope before discharging the spinning dope (filtration process) and/or a process of defoaming the spinning dope before discharging (defoaming process).

(Evaluation of fiber diameter of artificial structural protein fiber)

The fiber diameter can be calculated by the following equation on the assumption that the cross-sectional shape is a circle.

$$\text{Fiber diameter } [\mu\text{m}] = \{\text{average fineness } [\text{m/g}]/(\text{density of artificial structural protein } [\text{g/cm}^3] \times \pi)\}^{1/2}$$

The average fineness of fibers can be measured by the following procedure.

A fiber bundle is randomly sampled, cut into a length of 90 cm, and conditioned in an environment of a temperature of 20°C and a relative humidity of 65% for 12 hours or more. After conditioning, the mass of the fiber bundle is measured to calculate the average fineness, which is converted into the average fineness per single fiber (the number of samples = 5). The number of constituent fibers in the fiber bundle may be appropriately selected according to production conditions, and may be, for example, 1,000 (multifilaments composed of 1,000 single yarns).

(Evaluation of mechanical properties of artificial structural protein fiber)

The elongation and stress of an artificial structural protein fiber are measured according to JIS L 1013 using a 3345 series tensile tester manufactured by Instron Corporation. The test may be performed under conditions of a test

length of 300 mm and a test speed of 300 mm/min in an environment of a temperature of 20°C and a relative humidity of 65%. The load cell capacity may be appropriately selected according to the fineness of the fiber. The measured value may be calculated, for example, as the average value of the number of samples n = 5.

(Evaluation of shrinkage of artificial structural protein fiber)

The artificial structural protein fiber has characteristics of shrinking by being brought into contact (wetting) with water of less than the boiling point. Preferably, such a shrinkage is as little as possible in the artificial structural protein fiber. The shrinkage can be evaluated by using the shrinkage ratio as an indicator obtained by the following method.

A plurality of numbers of artificial structural protein fibers having a length of about 30 cm are bundled to form a fiber bundle with a fineness of 150 denier. A 0.8 g-lead weight is attached to this fiber bundle, and the fiber bundle is made shrunk by immersing the fiber bundle in this state in water at 40°C for 90 seconds. Then, each fiber bundle is taken out from the water, and dried with the 0.8 g-lead weight attached thereto. Then, the length of each fiber bundle after drying is measured. The shrinkage ratio is calculated according to the following equation. Note that $L_0$ represents the length of a fiber before contact with water (after spinning) (herein, 30 cm), and $L_D$ represents the length of a fiber after shrinkage (dried fiber after impregnation treatment with water).

$$\text{Shrinkage ratio } [\%] = \{1-(L_D/L_0)\} \times 100$$

[Product]

The protein fiber according to the present embodiment can be applied, as fibers (long fiber, short fiber, monofilament, or multifilament, for example) or yarns (spun yarn, twisted yarn, false-twisted yarn, processed yarn, combined filament yarn, or blended yarn, for example), to a woven fabric, a knitted fabric, a braided fabric, or a fabric such as a non-woven fabric, paper, and cotton, and the like. Also, the protein fiber according to the present embodiment can be applied to high strength applications such as ropes, surgical sutures, hemostatics, flexible stops for electrical components, and physiologically active materials for implantation (for example, artificial ligament and aortic band). These can be produced by a publicly known method.

Examples

Hereinafter, the present invention will be described more specifically based on Examples. However, the present invention is not limited to the following Examples.

[Production of artificial structural protein]

(1) Production of expression vector

An artificial structural protein (modified fibroin PRT966) having the SEQ ID NO: 44 was designed based on the base sequence and amino acid sequence of fibroin (GenBank Accession No.: P46804.1, GI: 1174415) derived from *Nephila clavipes*. Incidentally, the amino acid sequence set forth in SEQ ID NO: 44 has an amino acid sequence obtained by substituting all QQs with VF, substituting the remaining Q with I in the amino acid sequence set forth in SEQ ID NO: 9 (amino acid sequence before the amino acid sequence set forth in SEQ ID NO: 42 is added to the C-terminal thereof), for the purpose of improving hydrophobicity, and further adding the amino acid sequence set forth in SEQ ID NO: 12 to the N-terminal thereof.

Next, a nucleic acid encoding the designed artificial structural protein PRT966 having the amino acid sequence of SEQ ID NO: 44 was synthesized. In each of the nucleic acids, an NdeI site was added to the 5' end thereof, and an EcoRI site was added downstream of the stop codon thereof. The nucleic acid was each cloned into a cloning vector (pUC118). Then, the nucleic acid was excised by restriction enzyme treatment with NdeI and EcoRI, and then recombined into a protein expression vector pET-22b(+), thus obtaining an expression vector.

(2) Expression of artificial structural protein

*Escherichia coli* BLR(DE3) was transformed with the expression vector obtained in (1). The transformed *Escherichia coli* was cultured in 2 mL of an LB culture medium containing ampicillin for 15 hours. The culture solution was added to 100 mL of a seed culture medium containing ampicillin (Table 6) so that the $OD_{600}$ reached 0.005. The temperature of the culture solution was maintained at 30°C, and the flask culture was performed (for about 15 hours) until

the $OD_{600}$ reached 5, thus obtaining a seed culture solution.

[Table 6]

| Seed culture medium | |
|---|---|
| Reagent | Concentration (g/L) |
| Glucose | 5.0 |
| $KH_2PO_4$ | 4.0 |
| $K_2HPO_4$ | 9.3 |
| Yeast Extract | 6.0 |
| Ampicillin | 0.1 |

The seed culture solution was added to a jar fermenter to which 500 mL of a production medium (Table 7) has been added so that the $OD_{600}$ reached 0.05. Culture was performed while maintaining the temperature of the culture solution at 37°C and keeping the pH constant at 6.9. Further, the dissolved oxygen concentration in the culture solution was maintained at 20% of the dissolved oxygen saturation concentration.

[Table 7]

| Production medium | |
|---|---|
| Reagent | Concentration (g/L) |
| Glucose | 12.0 |
| $KH_2PO_4$ | 9.0 |
| $MgSO_4 \cdot 7H_2O$ | 2.4 |
| Yeast Extract | 15 |
| $FeSO_4 \cdot 7H_2O$ | 0.04 |
| $MnSO_4 \cdot 5H_2O$ | 0.04 |
| $CaCl_2 \cdot 2H_2O$ | 0.04 |
| GD-113 (antifoaming agent) | 0.1 (ml/L) |

Immediately after glucose in the production medium was completely consumed, a feed solution (455 g/1 L of glucose, 120 g/1 L of Yeast Extract) was added at a rate of 1 mL/min. Culture was performed while maintaining the temperature of the culture solution at 37°C and keeping the pH constant at 6.9. Further, the dissolved oxygen concentration in the culture solution was maintained at 20% of the dissolved oxygen saturation concentration, and culture was performed for 20 hours. Thereafter, 1 M isopropyl-β-thiogalactopyranoside (IPTG) was added to the culture solution so that the final concentration thereof was 1 mM, thus inducing the expression of the artificial structural protein. 20 hours after the addition of IPTG, the culture solution was centrifuged to recover bacterial cells. SDS-PAGE was performed using the bacterial cells prepared from the culture solutions before and after the addition of IPTG, and the expression of a desired artificial structural protein was confirmed by the appearance of a band of a desired artificial structural protein size depending on the addition of IPTG.

(3) Purification of artificial structural protein

The bacterial cells recovered 2 hours after the addition of IPTG were washed with 20 mM Tris-HCl buffer (pH 7.4). The bacterial cells after washing were suspended in 20 mM Tris-HCl buffer (pH 7.4) containing about 1 mM PMSF, and the cells were disrupted with a high-pressure homogenizer (manufactured by GEA Niro Soavi). The disrupted cells were centrifuged to obtain a precipitate. The obtained precipitate was washed with 20 mM Tris-HCl buffer (pH 7.4) until the purity of the precipitate became high. The precipitate after washing was suspended in 8 M guanidine buffer (8 M guanidine hydrochloride, 10 mM sodium dihydrogen phosphate, 20 mM NaCl, 1 mM Tris-HCl, pH 7.0) so that the concentration thereof was 100 mg/mL, and dissolved by stirring with a stirrer for 30 minutes at 60°C. After dissolution, dialysis was performed with water using a dialysis tube (cellulose tube 36/32, manufactured by Sanko Junyaku Co., Ltd.). The white aggregated protein obtained after dialysis was collected by centrifugation, moisture was removed with a lyophilizer, and a lyophilized powder was collected to obtain an artificial structural protein (fibroin PRT966).

[Evaluation of fiber-forming property]

(1) Preparation of dope solution

First, 26% by mass of a modified fibroin (PRT966) obtained in the production process of the artificial structural protein and 74% by mass of formic acid as a dissolving solvent (manufactured by Asahi Chemical Co., Ltd., purity: 98%) were mixed, and dissolved by heating the mixture by an aluminum block heater set at 70°C for 1 hour with stirring. The obtained solution was defoamed by filtration with a metal filter having an opening of 1 μm, and thus a dope solution was obtained.

(2) Discharge test of dope solution

The dope solution obtained in (1) was charged into a 10 ml-syringe, and then discharged from a nozzle having a nozzle diameter of 0.2 μm into the coagulation liquid, to coagulate the modified fibroin at room temperature. The coagulated raw fiber was wound up at a linear velocity of 2.39 m/min. The obtained raw fiber was observed, and the fiber-forming property was visually determined. The extrusion speed of the dope solution was 0.075 ml/min. The type of the coagulation liquid used is as shown in Table 8. Incidentally, the brackish water is collected from the estuary in Sakata city, Yamagata prefecture, and the sea water is collected from the ocean in Kamo city, Yamagata prefecture. The concentration of brackish water and sea water [wt%] indicates an approximate value of the concentration of the entire solutes. The mixed solutions of Test Examples 26 to 28 are a solution prepared based on an assumption that formic acid being in contact with the sodium chloride aqueous solution in the spinning dope is dissolved in the aqueous solution, and the proportion of the total mass of the coagulation liquid (mixed solution) is made such that the content of the sodium chloride aqueous solution is 60% by mass to 80% by mass, and the content of the formic acid is 20% by mass to 40% by mass. In the formic acid aqueous solution of Test Example 29, the proportion of the total mass (mixed solution) of the coagulation liquid was 80% by mass of water and 20% by mass of formic acid.

The evaluation result of the fiber-forming property was shown in Table 8. The evaluation criterion of the fiber-forming property is as follows.

⊙: Fiber is formed. The obtained fiber is flexible and homogeneous.
O: Fiber is formed. The obtained fiber is flexible.
△: Fiber is formed.
×: Fiber is not formed.

[Table 8]

| No. | Coagulation liquid | Concentration [% by mass] | pH | Fiber-forming property |
|---|---|---|---|---|
| Test Example 1 | Water | 100 | 7.22 | ○ |
| Test Example 2 | Citric acid monohydrate aqueous solution | 10 | 1.35 | ○ |
| Test Example 3 | | 20 | 1.09 | ○ |
| Test Example 4 | Sodium bicarbonate aqueous solution | 2.5 | 8.4 | ⊙ |
| Test Example 5 | Sodium formate aqueous solution | 5 | 7.36 | ⊙ |
| Test Example 6 | | 10 | 7.95 | ⊙ |
| Test Example 7 | | 20 | 8.42 | ⊙ |
| Test Example 8 | Sodium acetate | 10 | 8.78 | ⊙ |
| Test Example 9 | | 20 | 9.3 | ⊙ |
| Test Example 10 | Sodium citrate | 10 | 7.84 | ⊙ |
| Test Example 11 | | 20 | 7.7 | ⊙ |
| Test Example 12 | Potassium chloride aqueous solution | 6.5 | - | ⊙ |

(continued)

| No. | Coagulation liquid | Concentration [% by mass] | pH | Fiber-forming property |
|---|---|---|---|---|
| Test Example 13 | Sodium chloride aqueous solution | 5 | 5.97 | ⊙ |
| Test Example 14 | | 10 | 6.88 | ⊙ |
| Test Example 15 | | 15 | 6.55 | ⊙ |
| Test Example 16 | | 20 | 6.2 | ⊙ |
| Test Example 17 | Calcium chloride | 10 | 8.62 | ⊙ |
| Test Example 18 | | 20 | 8.71 | ⊙ |
| Test Example 19 | Sodium sulfate aqueous solution | 10 | 7.09 | ⊙ |
| Test Example 20 | | 20 | 6.78 | ⊙ |
| Test Example 21 | Ammonium sulfate aqueous solution | 5 | 4.89 | ⊙ |
| Test Example 22 | | 10 | 4.83 | ⊙ |
| Test Example 23 | | 20 | 4.75 | ⊙ |
| Test Example 24 | Buffer (1.5 M potassium dihydrogen phosphate and 1.5 M dipotassium hydrogen phosphate) | 20 | 7.5 | ⊙ |
| Test Example 25 | Brackish water | 1.6 | - | ⊙ |
| Test Example 26 | Sea water | 3.0 | - | ⊙ |
| Test Example 27 | Formic acid aqueous solution | 20 | 0.98 | ○ |
| Test Example 28 | Mixed solution (0.9 M sodium chloride aqueous solution: formic acid = 80 : 20) | - | 1.11 | ⊙ |
| Test Example 29 | Mixed solution (0.9 M sodium chloride aqueous solution: formic acid = 70 : 30) | - | 0.87 | ⊙ |
| Test Example 30 | Mixed solution (0.9 M sodium chloride aqueous solution: formic acid = 60 : 40) | - | 0.57 | ⊙ |
| Reference Example | Methanol | 100 | - | ○ |

As shown in Table 8, in a case of using any of water, an acid aqueous solution, a salt aqueous solution, and a mixed solution, flexible fibers could be formed (Test Examples 1 to 26). In a case of using a salt aqueous solution as a coagulation liquid, flexible and homogeneous fibers could be formed, and an extremely good fiber-forming property was shown (Test Examples 4 to 26). In particular, a large amount of production cost can be reduced by using, as a coagulation liquid, water, a sodium sulfate aqueous solution, a sodium chloride aqueous solution, brackish water, and sea water, which are abundant and inexpensive resources. The result also shows that, even in a case where a mixed aqueous solution of the organic solvent and the coagulation liquid is used as the coagulation liquid, flexible fibers could be formed (Test Examples 27 to 30). In particular, in a case where a coagulation liquid in which formic acid has been dissolved was a sodium chloride aqueous solution, flexible and homogeneous fibers could be formed (Test Examples 28 to 30).

[Production and evaluation of artificial structural protein fiber]

<Examples 1 to 24>

(1) Preparation of spinning dope (dope solution)

First, 30% by mass of an artificial structural protein (fibroin PRT966) obtained in the production process of the

artificial structural protein and 70% by mass of formic acid as a dissolving solvent (manufactured by Asahi Chemical Co., Ltd., purity: 98%) were mixed, and dissolved by heating the mixture by an aluminum block heater set at 40°C for 1 hour with stirring. The obtained solution was defoamed by filtration with a metal filter having an opening of 1 μm, and thus a dope solution was obtained.

(2) Wet spinning

The prepared dope solution was charged in a reserve tank, and discharged from a spinning nozzle (spinneret) having 200 holes into a coagulation bath using a gear pump to form a thread (original yarn). The take-up speed of the thread was a constant value. Then, the coagulated original yarn was drawn in a water washing bath. After washing and drawing in the water washing bath, the obtained yarn was dried by using a dry heat plate, and the resulting artificial structural protein fiber (modified fibroin fiber) was wound up by a winder. Conditions of the wet spinning were as follows. The values of the coagulation liquid and the bath draft used were shown in Table 9.

Hole diameter of spinneret: 0.05 mm
Temperature of coagulation liquid: 50°C
Temperature of water washing bath: 40°C
Temperature of drawing bath: 60°C
Total draw ratio: 4.8 times
Dry temperature: 60°C

(3) Evaluation of discharge stability

The evaluation results of the discharge stability of each coagulation liquid are shown in Table 9. The discharge stability was evaluated by setting the take-up speed to be constant and changing the value of the discharge linear velocity. A sodium chloride aqueous solution was used as a chloride aqueous solution, a sodium sulfate aqueous solution was used as a sulfate, and a sodium citrate aqueous solution was used as a carboxylate. Evaluation criteria of the discharge stability are as follows.

⊙: There is no slack in the thread immediately after discharge, and fibers can be passed through.
O: There is slight slack in the thread immediately after discharge, but fibers can be passed through.
△: The thread immediately after discharged has a large slack, but fibers can be passed through.
×: The slack of the thread immediately after discharge is large, and fibers cannot be passed through.

[Table 9]

| | Type of coagulation liquid | | | | | | | |
|---|---|---|---|---|---|---|---|---|
| Bath draft [time] | Water | | 3.0% by mass sodium chloride aqueous solution | | 12% by mass sodium sulfate aqueous solution | | 21% by mass sodium citrate aqueous solution | |
| 2.7 | Example 1 | ⊙ | Example 7 | ⊙ | Example 13 | ⊙ | Example 19 | ⊙ |
| 2.0 | Example 2 | ⊙ | Example 8 | ⊙ | Example 14 | ⊙ | Example 20 | ⊙ |
| 1.6 | Example 3 | ○ | Example 9 | ○ | Example 15 | ⊙ | Example 21 | ⊙ |
| 1.3 | Example 4 | ○ | Example 10 | ○ | Example 16 | ⊙ | Example 22 | ⊙ |
| 1.1 | Example 5 | △ | Example 11 | ○ | Example 17 | ⊙ | Example 23 | ⊙ |
| 1.0 | Example 6 | △× | Example 12 | Δ | Example 18 | ⊙ | Example 24 | ⊙ |

As shown in Table 9, even in a case where water was used as the coagulation liquid, fibers could be formed in a range of bath draft of 1.0 to 2.7 (Examples 1 to 6), but in a case where the coagulation liquid was a salt aqueous solution (Examples 7 to 24), the discharge stability was further improved. In particular, in a case where a sulfate aqueous solution (sodium sulfate aqueous solution, Examples 13 to 18) and a carboxylic acid aqueous solution (sodium citrate aqueous solution, Examples 19 to 24) were used for the coagulation liquid, the discharge stability was superior, and the discharge linear velocity could be further increased, so that the productivity could be improved.

<Examples 25 (reference example) and 26 to 46>

(4) Evaluation of spinning stability

An artificial structural protein fiber was each produced by performing wet spinning in the same procedure as in Examples 1 to 24 except that a spinneret (spinning nozzle) having 1,000 holes was used and conditions of the wet spinning were set to the hole diameter of the spinneret and the value of the bath draft shown in Table 10. The spinning stability of the obtained artificial structural protein fiber was evaluated by slack and breakage of the thread, and the results are shown in Table 10.

Coagulation liquid: a mixed solution of 14.4% by mass of sodium sulfate, 65.6% by mass of water (80% by mass of a 18% by mass sodium sulfate aqueous solution), and 20% by mass of formic acid
Temperature of coagulation liquid: 40°C
Total draw ratio: 5 times

[Table 10]

| | Example 25 | Example 26 | Example 27 | Example 28 | Example 29 | Example 30 |
|---|---|---|---|---|---|---|
| Hole diameter of spinneret [mm] | 0.04 | | | | | |
| Bath draft [time] | 0.5 | 0.7 | 1 | 3.3 | 5 | 10 |
| Slack of thread | None | None | None | None | None | None |
| Breakage of yarn | None | None | None | None | None | None |
| | Example 31 | Example 32 | Example 33 | Example 34 | Example 35 | |
| Hole diameter of spinneret [mm] | 0.06 | | | | | |
| Bath draft [time] | 0.7 | 0.9 | 1.3 | 6.7 | 10 | |
| Slack of thread | None | None | None | None | None | |
| Breakage of yarn | None | None | None | None | None | |
| | Example 36 | Example 37 | Example 38 | Example 39 | Example 40 | |
| Hole diameter of spinneret [mm] | 0.08 | | | | | |
| Bath draft [time] | 1.4 | 2.0 | 6.7 | 10 | 20 | |
| Slack of thread | None | None | None | None | None | |
| Breakage of yarn | None | None | None | None | None | |
| | Example 41 | Example 42 | Example 43 | Example 44 | Example 45 | Example 46 |
| Hole diameter of spinneret [mm] | 0.1 | | | | | |
| Bath draft [time] | 1.8 | 2.3 | 2.8 | 10 | 14 | 16 |
| Slack of thread | None | None | None | None | None | None |
| Breakage of yarn | None | None | None | None | None | None |

As shown in Table 10, in the wet spinning using a spinneret having a hole diameter of 0.04 mm (Examples 25 to 30) and 0.06 mm (Examples 31 to 35), when the dope solution was discharged to form a thread, fibers could be stably produced without causing slack and breakage of the thread over a wide range of bath draft of 0.5 to 10 times (Examples 25 to 30) and 0.7 to 10 times (Examples 31 to 35).

Regarding the range of bath draft of less than 0.5 and more than 10 times (hole diameter: 0.04 mm) and the range of bath draft of less than 0.7 and more than 10 times (hole diameter: 0.06 mm), although the test could not be carried out due to the lower limit on the facility related to the discharge speed and the take-up speed, it is considered that fibers can be

similarly produced.

As shown in Table 10, in the wet spinning using a spinneret having a hole diameter of 0.08 mm (Examples 36 to 40) and 0.1 mm (Examples 41 to 46), when the dope solution was discharged to form a thread, fibers could be stably produced without causing slack and breakage of the thread over a wide range of bath draft of 1.4 to 20 times (Examples 36 to 40) and 1.8 to 16 times (Examples 41 to 46). Regarding the range of bath draft of less than 1.4 and more than 20 times (hole diameter: 0.08 mm) and the range of bath draft of less than 1.8 and more than 16 times (hole diameter: 0.08 mm), although the test could not be carried out due to the lower limit on the facility related to the discharge speed and the take-up speed, it is considered that fibers can be similarly produced.

As described above, the spinning stability was obtained in a wide range of bath draft (0.5 to 20). Since the value of the bath draft can be adjusted in a wide range, it is possible to perform wet spinning using a spinneret having a larger hole diameter according to a desired fiber diameter of the fiber. The results show that the productivity can be further improved by the present production method.

<Examples 47 to 56>

(5) Wet spinning

<Examples 47 to 48>

An artificial structural protein fiber (modified fibroin fiber) was each produced by performing wet spinning in the same manner as in Examples 25 to 30 except that the hole diameter of the spinneret (spinning nozzle) and the bath draft were set to the values in Table 11, and a 11.9% by mass sodium sulfate aqueous solution was used as the coagulation liquid.

<Examples 49 to 56>

An artificial structural protein fiber (modified fibroin fiber) was each produced by performing wet spinning in the same manner as in Examples 25 to 30 except that the hole diameter of the spinneret and the bath draft were set to the values in Table 11.

(6) Evaluation of physical properties

The physical properties of the artificial structural protein fibers obtained in the above (5) were evaluated based on the following fiber diameter evaluation, mechanical property evaluation, and fiber shrinkage evaluation. The results are shown in Tables 11 and 12.

(Fiber diameter evaluation)

The fiber diameter was calculated by the following equation on the assumption that the cross-sectional shape was a circle.

$$\text{Fiber diameter } [\mu\text{m}] = \{\text{average fineness } [\text{m/g}]/(\text{density of artificial structural protein } [\text{g/cm}^3] \times \pi)\}^{1/2}$$

The average fineness of fibers was measured by the following procedure. A fiber bundle was randomly sampled, cut into a length of 90 cm, and conditioned in an environment of a temperature of 20°C and a relative humidity of 65% for 12 hours or more. After conditioning, the mass of the fiber bundle was measured to calculate the average fineness, which was converted into the average fineness per single fiber. The number of samples was n = 5. The density of the artificial structural protein (modified fibroin PRT966) was 1.34 [g/cm$^3$].

(Evaluation of mechanical properties)

The elongation and stress of an artificial structural protein fiber were measured according to JIS L 1013 using a 3345 series tensile tester manufactured by Instron Corporation. The test was performed under conditions of a load cell capacity of 50 N, a test length of 300 mm, and a test speed of 300 mm/min in an environment of a temperature of 20°C and a relative humidity of 65%. The measured value was calculated as the average value of the number of samples n = 5.

(Evaluation of fiber shrinkage)

The shrinkage was evaluated using the shrinkage ratio determined by the following method as an index. A plurality of numbers of artificial structural protein fibers having a length of about 30 cm are bundled to form a fiber bundle with a fineness of 150 denier. A 0.8 g-lead weight was attached to this fiber bundle, and the fiber bundle was made shrunk by immersing the fiber bundle in this state in water at 40°C for 90 seconds. Then, each fiber bundle was taken out from the water, and dried with the 0.8 g-lead weight attached thereto. Then, the length of each fiber bundle after drying was measured. The shrinkage ratio was calculated according to the following equation with the number of samples as n = 2.

$$\text{Shrinkage ratio } [\%] = \{1-(L_D/L_0)\} \times 100$$

Note that $L_0$ represents the length of a fiber before contact with water (after spinning) (herein, 30 cm), and $L_D$ represents the length of a fiber after shrinkage (dried fiber after impregnation treatment with water).

<Comparative Example>

An artificial structural protein fiber (modified fibroin fiber) was each produced by performing wet spinning using a spinneret having a hole diameter of 0.04 mm in the same manner as in Examples 25 to 30 except that the value of the bath draft was set to 0.4 times. The results of physical property evaluation of the obtained fibers are shown in Tables 11 and 12.

[Table 11]

| | Example 56 | Example 55 | Example 54 | Example 53 | Example 52 | Example 51 |
|---|---|---|---|---|---|---|
| Hole diameter of spinneret [μm] | 0.08 | | | | 0.06 | |
| Bath draft [time] | 6.4 | 4.0 | 3.2 | 2.6 | 1.6 | 1.4 |
| Total draw ratio [time] | 5 | 5 | 5 | 5 | 5 | 5 |
| Average fiber diameter [μm] | 9.7 | 10.1 | 11.2 | 12.2 | 11.7 | 12.9 |
| Relative value of elongation [%] | 75 | 108 | 187 | 187 | 199 | 162 |
| Relative value of stress [%] | 184 | 179 | 164 | 175 | 173 | 156 |
| | Example 50 | Example 49 | Example 48 | Example 47 | Comparative Example | |
| Hole diameter of spinneret [μm] | 0.05 | | 0.04 | | 0.04 | |
| Bath draft [time] | 1.6 | 0.8 | 1.6 | 0.8 | 0.4 | |
| Total draw ratio [time] | 5 | 5 | 5 | 5 | 5 | |
| Average fiber diameter [μm] | 9.1 | 10 | 8.1 | 11.4 | 16 | |
| Relative value of elongation [%] | 228 | 180 | 379 | 255 | 100 | |
| Relative value of stress [%] | 115 | 111 | 113 | 94 | 100 | |

[Table 12]

| | Example 48 | Example 47 | Comparative Example |
|---|---|---|---|
| Bath draft [time] | 1.6 | 0.8 | 0.4 |
| Total draw ratio [time] | 5 | 5 | 5 |
| Relative value of shrinkage ratio [%] | 66 | 46 | 100 |

As shown in Table 11, in the artificial structural protein fibers in which the bath draft was increased to 0.8 to 6.4 times (Examples 47 to 56), fibers having a reduced fiber diameter and further having a stress equal to or higher than that of the artificial structural protein fibers in which the bath draft was decreased to 0.4 times (Comparative Example) were obtained, and unexpected excellent results were obtained. In particular, in a case where the bath draft was 2.0 times, fibers having a small diameter of 8 μm were obtained and the stress was improved as compared with Comparative Example.

Further, as shown in Table 12, the effect of reducing the shrinkage ratio with respect to water was obtained, and an extremely excellent effect was obtained (Example 48). In addition, in a case where the bath draft was 6.4 times, the fiber diameter was reduced as compared with Comparative Example, and the stress value was most improved to 184% (Example 56). The above results show that when the bath draft was more than 0.4, fibers having a stress value equal to or higher than those of fibers with reduced diameter were obtained. The results also show that the effect of further improving the stress is exhibited by setting the bath draft to more than 0.8. The relative values of the stress, the elongation, and the shrinkage ratio in Tables 11 and 12 are relative values when the values of the stress, the elongation, and the shrinkage ratio of the artificial structural protein fiber of Comparative Example are respectively 100 [%]. Reference Signs List

- 1 Extrusion apparatus
- 2 Undrawn yarn producing apparatus
- 3 Wet heat drawing apparatus
- 4 Drying apparatus
- 6 Spinning dope
- 10 Spinning apparatus
- 20 Coagulation bath
- 21 Washing bath
- 36 Protein fiber

[Sequence listing]

## Claims

**1.** A method for producing an artificial structural protein fiber by a wet spinning method, the method comprising a coagulation step of discharging a spinning dope containing an artificial structural protein and an organic solvent from a spinneret into a coagulation liquid to coagulate the artificial structural protein, wherein a bath draft in the coagulation step is 0.7 to 20.

**2.** The method according to claim 1, wherein the coagulation liquid contains water or an aqueous solution having a pH of 0.25 or more and 10.00 or less.

**3.** The method according to claim 2, wherein a content of the water or aqueous solution having a pH of 0.25 or more and 10.00 or less in the coagulation liquid is 70% by mass or more based on 100% by mass of a total amount of the coagulation liquid.

**4.** The method according to claim 2 or 3, wherein the aqueous solution is at least one type selected from the group consisting of a sulfate aqueous solution, a chloride aqueous solution, a carboxylate aqueous solution, a hydrogen phosphate aqueous solution, a bicarbonate aqueous solution, brackish water, and sea water.

**5.** The method according to any one of claims 2 to 4, wherein the aqueous solution is at least one type selected from the group consisting of a sodium chloride aqueous solution, a sodium sulfate aqueous solution, and a sodium citrate aqueous solution.

**6.** The method according to any one of claims 1 to 5, wherein a content of the artificial structural protein in the spinning dope is more than 10% by mass and 50% by mass or less based on 100% by mass of a total amount of the spinning dope.

**7.** The method according to any one of claims 1 to 6, wherein an average hydropathy index of the artificial structural protein is more than -0.8.

**8.** The method according to any one of claims 1 to 7, wherein the artificial structural protein contains at least one type selected from the group consisting of a spider silk fibroin, a silk fibroin, and a keratin protein.

**9.** The method according to any one of claims 1 to 8, wherein the artificial structural protein is a spider silk fibroin.

10. The method according to any one of claims 1 to 9, wherein the artificial structural protein is a modified spider silk fibroin.

11. The method according to any one of claims 1 to 10, wherein the organic solvent in the spinning dope is at least one type selected from the group consisting of formic acid and hexafluoroisopropanol.

12. The method according to any one of claims 1 to 11, wherein the coagulation liquid contains the organic solvent, and a content of the organic solvent in the coagulation liquid is 10% by mass or more and 30% by mass or less based on 100% by mass of a total amount of the coagulation liquid.

## Patentansprüche

1. Verfahren zum Produzieren einer künstlichen Strukturproteinfaser durch ein Nassspinnverfahren, wobei das Verfahren einen Koagulationsschritt zum Austragen einer Spinnlösung, die ein künstliches Strukturprotein und ein organisches Lösungsmittel enthält, aus einer Spinndüse in eine Koagulationsflüssigkeit zum Koagulieren des künstlichen Strukturproteins umfasst, wobei ein Badverzug in dem Koagulationsschritt 0,7 bis 20 beträgt.

2. Verfahren nach Anspruch 1, wobei die Koagulationsflüssigkeit Wasser oder eine wässrige Lösung mit einem pH-Wert von 0,25 oder mehr und 10,00 oder weniger enthält.

3. Verfahren nach Anspruch 2, wobei ein Gehalt des Wassers oder der wässrigen Lösung mit einem pH-Wert von 0,25 oder mehr und 10,00 oder weniger in der Koagulationsflüssigkeit 70 Masse-% oder mehr basierend auf 100 Masse-% einer Gesamtmenge der Koagulationsflüssigkeit beträgt.

4. Verfahren nach Anspruch 2 oder 3, wobei die wässrige Lösung mindestens ein Typ ist, ausgewählt aus der Gruppe bestehend aus einer wässrigen Sulfatlösung, einer wässrigen Chloridlösung, einer wässrigen Carboxylatlösung, einer wässrigen Hydrogenphosphatlösung, einer wässrigen Hydrogencarbonatlösung, Brackwasser und Meerwasser.

5. Verfahren nach einem der Ansprüche 2 bis 4, wobei die wässrige Lösung mindestens ein Typ ist, ausgewählt aus der Gruppe bestehend aus einer wässrigen Natriumchloridlösung, einer wässrigen Natriumsulfatlösung und einer wässrigen Natriumcitratlösung.

6. Verfahren nach einem der Ansprüche 1 bis 5, wobei ein Gehalt des künstlichen Strukturproteins in der Spinnlösung mehr als 10 Masse-% und 50 Masse-% oder weniger basierend auf 100 Masse-% einer Gesamtmenge der Spinnlösung beträgt.

7. Verfahren nach einem der Ansprüche 1 bis 6, wobei ein durchschnittlicher Hydropathie-Index des künstlichen Strukturproteins mehr als -0,8 beträgt.

8. Verfahren nach einem der Ansprüche 1 bis 7, wobei das künstliche Strukturprotein mindestens einen Typ enthält, ausgewählt aus der Gruppe bestehend aus einem Spinnenseidenfibroin, einem Seidenfibroin und einem Keratinprotein.

9. Verfahren nach einem der Ansprüche 1 bis 8, wobei das künstliche Strukturprotein ein Spinnenseidenfibroin ist.

10. Verfahren nach einem der Ansprüche 1 bis 9, wobei das künstliche Strukturprotein ein modifiziertes Spinnenseidenfibroin ist.

11. Verfahren nach einem der Ansprüche 1 bis 10, wobei das organische Lösungsmittel in der Spinnlösung mindestens ein Typ ist, ausgewählt aus der Gruppe bestehend aus Ameisensäure und Hexafluorisopropanol.

12. Verfahren nach einem der Ansprüche 1 bis 11, wobei die Koagulationsflüssigkeit das organische Lösungsmittel enthält und ein Gehalt des organischen Lösungsmittels in der Koagulationsflüssigkeit 10 Masse-% oder mehr und 30 Masse-% oder weniger basierend auf 100 Masse-% einer Gesamtmenge der Koagulationsflüssigkeit beträgt.

## Revendications

1. Procédé permettant la production d'une fibre de protéine structurale artificielle par un procédé de filage humide, le procédé comprenant une étape de coagulation consistant à décharger une solution à filer contenant une protéine structurale artificielle et un solvant organique à partir d'une filière dans un liquide de coagulation afin de coaguler la protéine structurale artificielle, dans lequel un tirage dans le bain lors de l'étape de coagulation est de 0,7 à 20.

2. Procédé selon la revendication 1, dans lequel le liquide de coagulation contient de l'eau ou une solution aqueuse ayant un pH de 0,25 ou plus et de 10,00 ou moins.

3. Procédé selon la revendication 2, dans lequel une teneur en eau ou en solution aqueuse ayant un pH de 0,25 ou plus et de 10,00 ou moins dans le liquide de coagulation est de 70 % en masse ou plus sur la base de 100 % en masse d'une quantité totale du liquide de coagulation.

4. Procédé selon l'une des revendications 2 ou 3, dans lequel la solution aqueuse est au moins un type choisi dans le groupe constitué d'une solution aqueuse de sulfate, d'une solution aqueuse de chlorure, d'une solution aqueuse de carboxylate, d'une solution aqueuse d'hydrogénophosphate, d'une solution aqueuse de bicarbonate, d'eau saumâtre et d'eau de mer.

5. Procédé selon l'une quelconque des revendications 2 à 4, dans lequel la solution aqueuse est au moins un type choisi dans le groupe constitué d'une solution aqueuse de chlorure de sodium, d'une solution aqueuse de sulfate de sodium et d'une solution aqueuse de citrate de sodium.

6. Procédé selon l'une quelconque des revendications 1 à 5, dans lequel une teneur en protéine structurale artificielle dans la solution à filer est de plus de 10 % en masse et de 50 % en masse ou moins sur la base de 100 % en masse d'une quantité totale de la solution à filer.

7. Procédé selon l'une quelconque des revendications 1 à 6, dans lequel un indice d'hydropathie moyen de la protéine structurale artificielle est supérieur à -0,8.

8. Procédé selon l'une quelconque des revendications 1 à 7, dans lequel la protéine structurale artificielle contient au moins un type choisi dans le groupe constitué d'une fibroïne de soie d'araignée, d'une fibroïne de soie et d'une protéine de kératine.

9. Procédé selon l'une quelconque des revendications 1 à 8, dans lequel la protéine structurale artificielle est une fibroïne de soie d'araignée.

10. Procédé selon l'une quelconque des revendications 1 à 9, dans lequel la protéine structurale artificielle est une fibroïne de soie d'araignée modifiée.

11. Procédé selon l'une quelconque des revendications 1 à 10, dans lequel le solvant organique dans la solution à filer est au moins un type choisi dans le groupe constitué de l'acide formique et de l'hexafluoroisopropanol.

12. Procédé selon l'une quelconque des revendications 1 à 11, dans lequel le liquide de coagulation contient le solvant organique, et une teneur en solvant organique dans le liquide de coagulation est de 10 % en masse ou plus et de 30 % en masse ou moins sur la base de 100 % en masse d'une quantité totale du liquide de coagulation.

FIG. 1

AAAA
50AA
AAAA
100AA
AAAA
10AA
AAAA
20AA
AAAA
30AA
AAAA
PATTERN 1
PATTERN 2
PATTERN 3
PATTERN 4
.....

AAAA
30
4
4
AAAA
20
4
AAAA
10
AAAA
40
4
4
AAAA
50
4
4
AAAA

FIG. 2

REGION A
AAAA
50
GPGXX GPGXX
AAAA
40
GPGXX GPGXX
AAAA
10
AAAA
20
GPGXX
AAAA
30
GPGXX GPGXX
AAAA

FIG. 3

10
5
36
17
22
4
18g
18f
18e
14
12
3
21
13
18d
18c
18b
11
2
20
18a
9
7
6
8
1

FIG. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 5540166 B
- WO 2017131196 A
- JP 2014138877 A
- WO 2018164021 A1
- WO 2018034111 A1
- JP 2002238569 A

### Non-patent literature cited in the description

- *Nucleic Acid Res.*, 1982, vol. 10, 6487
- *Methods in Enzymology*, 1983, vol. 100, 448
- **KYTE J** ; **DOOLITTLE R**. A simple method for displaying the hydropathic character of a protein. *J. Mol. Biol.*, 1982, vol. 157, 105-132
- *Proc. Natl. Acad. Sci. USA*, 1972, vol. 69, 2110
- Molecular Cloning